# EUROPEAN PATENT APPLICATION

(11) **EP 4 803 541 A1**
(43) Date of publication of application: **09.09.2026**
(21) Application number: 24896684.8
(22) Date of filing: 29.11.2024
(51) Int. Cl.: C07K 16/26, C07K 14/61, C07K 19/00, C12P 21/08, G01N 33/577

(54) **ANTI-GH SINGLE-DOMAIN ANTIBODY AND USE THEREOF**

(30) Priority: 30.11.2023 CN 202311630170; 12.07.2024 CN 202410938901
(71) Applicant: Changchun Genescience Pharmaceutical Co., Ltd., Changchun, Jilin 130012 (CN)
(72) Inventor: WANG, Siqin, Changchun, Jilin 130012 (CN); LI, Shuang, Changchun, Jilin 130012 (CN); ZONG, Yi, Changchun, Jilin 130012 (CN); ZHANG, Rui, Changchun, Jilin 130012 (CN); LI, Jiajun, Changchun, Jilin 130012 (CN); LYU, Limin, Changchun, Jilin 130012 (CN); LI, Chunshu, Changchun, Jilin 130012 (CN); JIN, Lei, Changchun, Jilin 130012 (CN)
(74) Representative: E. Blum & Co. AG
(86) International application number: PCT/CN2024/135440
(87) International publication number: WO 2025/113599

(57) **Abstract**

Provided are a single-domain antibody specifically binding to a growth hormone (e.g., a human growth hormone) or an antigen-binding fragment thereof, a polypeptide construct and conjugate containing the single-domain antibody or the antigen-binding fragment thereof, a nucleic acid molecule encoding the single-domain antibody or the antigen-5 binding fragment thereof, a host cell containing same, and the related use. In addition, further provided is the use of the single-domain antibody or the antigen-binding fragment thereof, the polypeptide construct or the conjugate in the purification and/or detection of the growth hormone (e.g., the human growth hormone).

## Description

The present application is based on and claims priority to the CN patent application No. 202311630170.3 filed on November 30, 2023, and the CN patent application No. 202410938901.9 filed on July 12, 2024, the disclosures of which are hereby incorporated herein by reference in their entirety.

### TECHNICAL FIELD

The present invention relates to a single-domain antibody specifically binding to a growth hormone (e.g., a human growth hormone) or an antigen-binding fragment thereof, a polypeptide construct and conjugate containing the single-domain antibody or antigen-binding fragment thereof, a nucleic acid molecule encoding the single-domain antibody or antigen-binding fragment thereof, a host cell containing same, and the related use. In addition, the present invention relates to the use of the single-domain antibody or antigen-binding fragment thereof, the polypeptide construct or the conjugate in the purification and/or detection of the growth hormone (e.g., the human growth hormone).

### BACKGROUND

Human growth hormone (hGH) is a protein secreted by adenohypophyseal cells, a peptide hormone, a single-chain polypeptide hormone secreted by acidophilic cells in the anterior lobe of the human pituitary gland, and a peptide hormone consisting of 191 amino acids. Growth hormone, as a specific drug for treating dwarfism, has been used clinically since 1958. After it was made available for large-scale production in pharmaceutical factories in the 1980s, its clinical indications have been continuously expanded. It is widely used not only in promoting human height growth in clinical practice, but also in combating organ failure, improving nutrition in critically ill patients, treating cachexia (nutritional failure), fighting infection and inflammation, promoting wound and burn healing, and enhancing the body's immunity.

Nanobodies are a special type of antibodies derived from Camelidae animals. In 1993, Hamers-Casterman et al. demonstrated that naturally occurring heavy-chain antibodies devoid of light chains exist in Camelidae animals, which are referred to as heavy-chain antibodies. By cloning the variable region genes of heavy-chain antibodies, single-domain antibodies composed of only one heavy chain variable region can be obtained, which are designated as VHH antibodies. In the crystal structure of VHH antibodies, they have a diameter of only 2.5 nm and a length of 4 nm, and are therefore also referred to as nanobodies. Nanobodies are only one-tenth the size of traditional IgG antibodies and are the smallest naturally occurring fragments that can bind to antigens. In addition, nanobodies are easy to produce and can be used for the immunoaffinity chromatography purification of human GH, replacing the cumbersome traditional human GH purification methods.

With the optimization and iteration of developed immunoaffinity chromatography media for GH, higher requirements are imposed on the heat resistance and alkali resistance of ligand proteins on the media. Therefore, there is an urgent need to develop antibodies that specifically bind to GH and have excellent heat and alkali resistance for the purification and/or detection of GH.

### SUMMARY OF THE INVENTION

Through extensive research, the inventors of the present application have obtained a series of single-domain antibodies against growth hormones (e.g., human growth hormones). In particular, these single-domain antibodies, while possessing high binding activity to growth hormones (e.g., human growth hormones), also exhibit significantly superior heat and alkali resistance properties, making them advantageous for affinity purification of growth hormones (e.g., human growth hormones). For example, compared to existing anti-GH antibodies, immunoaffinity media prepared using the single-domain antibodies provided in the present application are easier to clean, have a longer shelf life, and can be stored at room temperature for an extended period. In addition, the single-domain antibody features low molecular weight and ease of production.

Based on this, the present application further provides a polypeptide construct or conjugate containing the single-domain antibody or antigen-binding fragment thereof, a nucleic acid molecule encoding the single-domain antibody or antigen-binding fragment thereof, a host cell containing same, and the related use.

Therefore, in a first aspect, the present application provides a single-domain antibody capable of specifically binding to a growth hormone (GH), or an antigen-binding fragment thereof. The single-domain antibody or antigen-binding fragment thereof comprises complementarity-determining regions (CDRs) and framework regions (FRs), and has one or more features selected from the group consisting of:
(i) the amino acid residue at position 102 is Y;
(ii) the amino acid residue at position 105 is Q;
(iii) the amino acid residue at position 48 is V;
(iv) the amino acid residue at position 11 is K;
(v) the amino acid residue at position 100g is F;
(vi) the amino acid residue at position 101 is D;
(vii) the amino acid residue at position 28 is T;
(viii) the amino acid residue at position 19 is T;
(ix) the amino acid residue at position 56 is K;
(x) the amino acid residue at position 100e is E;
(xi) the amino acid residue at position 55 is D;
(xii) the amino acid residue at position 76 is S;
(xiii) the amino acid residue at position 14 is P;
wherein the positions are defined by the kabat numbering system.

In some embodiments, the single-domain antibody or antigen-binding fragment thereof comprises CDR1, CDR2 and CDR3.

It is well known to those skilled in the art that the positions of amino acid residues in the antibody light chain variable region (VL) and heavy chain variable region (VH) are defined according to the kabat numbering system. For example, CDRs of antibody VH (or single-domain antibody VHH) domains can generally be defined as containing the following amino acids: amino acid residues corresponding to positions 31 to 35 or positions 31 to 35c (CDRH1), amino acid residues corresponding to positions 50 to 65 (CDRH2), and amino acid residues corresponding to positions 95 to 102 (CDRH3) in the heavy chain variable domain. See Kabat et al., Sequences of Proteins of Immunological Interest, 5th ed., Public Health Service, National Institutes of Health, Bethesda, MD. (1991), the full text of which is incorporated herein by reference.

In some embodiments, the single-domain antibody or antigen-binding fragment thereof has one or more features selected from the group consisting of:
(a) the amino acid residue at position 102 is Y;
(b) the amino acid residue at position 105 is Q;
(c) the amino acid residue at position 48 is V;
(d) the amino acid residue at position 100g is F;
(e) the amino acid residue at position 28 is T;
(f) the amino acid residue at position 19 is T;
(g) the amino acid residue at position 100e is E;
(h) the amino acid residue at position 55 is D;
(i) the amino acid residue at position 14 is P;
wherein the positions are defined by the kabat numbering system.

In some embodiments, the single-domain antibody or antigen-binding fragment thereof has features selected from the group consisting of:
(1) the amino acid residue at position 55 is D, and the amino acid residue at position 100e is E;
(2) the amino acid residue at position 28 is T, and the amino acid residue at position 55 is D;
(3) the amino acid residue at position 14 is P, and the amino acid residue at position 28 is T;
(4) the amino acid residue at position 14 is P, and the amino acid residue at position 105 is Q;
(5) the amino acid residue at position 14 is P, and the amino acid residue at position 19 is T;
(6) the amino acid residue at position 14 is P, and the amino acid residue at position 100e is E;
(7) the amino acid residue at position 55 is D, the amino acid residue at position 100e is E, and the amino acid residue at position 105 is Q;
(8) the amino acid residue at position 55 is D, the amino acid residue at position 100g is F, and the amino acid residue at position 102 is Y;
(9) the amino acid residue at position 55 is D, the amino acid residue at position 100e is E, and the amino acid residue at position 102 is Y;
(10) the amino acid residue at position 55 is D, and the amino acid residue at position 100g is F;
(11) the amino acid residue at position 55 is D, the amino acid residue at position 100e is E, and the amino acid residue at position 100g is F;
(12) the amino acid residue at position 55 is D, and the amino acid residue at position 102 is Y;
(13) the amino acid residue at position 48 is V, the amino acid residue at position 55 is D, and the amino acid residue at position 100e is E;
(14) the amino acid residue at position 55 is D, the amino acid residue at position 100e is E, and the amino acid residue at position 105 is Q;
(15) the amino acid residue at position 55 is D, the amino acid residue at position 100e is E, the amino acid residue at position 100g is F, and the amino acid residue at position 105 is Q;
wherein the positions are defined by the kabat numbering system.

In some embodiments, the single-domain antibody or antigen-binding fragment thereof has any one of the following CDR1, CDR2 and CDR3:
(1) CDR1 as set forth in SEQ ID NO: 42; CDR2 as set forth in SEQ ID NO: 43; and CDR3 as set forth in SEQ ID NO: 47;
(2) CDR1 as set forth in SEQ ID NO: 42; CDR2 as set forth in SEQ ID NO: 43; and CDR3 as set forth in SEQ ID NO: 48;
(3) CDR1 as set forth in SEQ ID NO: 42; CDR2 as set forth in SEQ ID NO: 43; and CDR3 as set forth in SEQ ID NO: 49;
(4) CDR1 as set forth in SEQ ID NO: 42; CDR2 as set forth in SEQ ID NO: 44; and CDR3 as set forth in SEQ ID NO: 46;
(5) CDR1 as set forth in SEQ ID NO: 42; CDR2 as set forth in SEQ ID NO: 43; and CDR3 as set forth in SEQ ID NO: 50;
(6) CDR1 as set forth in SEQ ID NO: 42; CDR2 as set forth in SEQ ID NO: 45; and CDR3 as set forth in SEQ ID NO: 46;
(7) CDR1 as set forth in SEQ ID NO: 42; CDR2 as set forth in SEQ ID NO: 45; and CDR3 as set forth in SEQ ID NO: 50;
(8) CDR1 as set forth in SEQ ID NO: 42; CDR2 as set forth in SEQ ID NO: 45; and CDR3 as set forth in SEQ ID NO: 51;
(9) CDR1 as set forth in SEQ ID NO: 42; CDR2 as set forth in SEQ ID NO: 45; and CDR3 as set forth in SEQ ID NO: 52;
(10) CDR1 as set forth in SEQ ID NO: 42; CDR2 as set forth in SEQ ID NO: 45; and CDR3 as set forth in SEQ ID NO: 48;
(11) CDR1 as set forth in SEQ ID NO: 42; CDR2 as set forth in SEQ ID NO: 45; and CDR3 as set forth in SEQ ID NO: 53;
   or,
(12) CDR1 as set forth in SEQ ID NO: 42; CDR2 as set forth in SEQ ID NO: 45; and CDR3 as set forth in SEQ ID NO: 47.

In some embodiments, compared with the sequence as set forth in SEQ ID NO: 37, the single-domain antibody has one or more substitution mutations selected from the group consisting of:
D102Y, P105Q, L48V, L11K, W100gF, V101D, P28T, R19T, T56K, R100eE, A55D, N76S, A14P;
wherein the positions of the mutations are defined by the kabat numbering system.

In some embodiments, compared with the sequence as set forth in SEQ ID NO: 37, the single-domain antibody has one or more substitution mutations selected from the group consisting of:
D102Y, P105Q, L48V, W100gF, P28T, R19T, R100eE, A55D, A14P;
wherein the positions of the mutations are defined by the kabat numbering system.

In some embodiments, compared with the sequence as set forth in SEQ ID NO: 37, the single-domain antibody has:
(1) substitution of A at position 55 with D, and substitution of R at position 100e with E (A55D and R100eE);
(2) substitution of P at position 28 with T, and substitution of A at position 55 with D (P28T and A55D);
(3) substitution of A at position 14 with P, and substitution of P at position 28 with T (A14P and P28T);
(4) substitution of A at position 14 with P, and substitution of P at position 105 with Q (A14P and P105Q);
(5) substitution of A at position 14 with P, and substitution of R at position 19 with T (A14P and R19T);
(6) substitution of A at position 14 with P, and substitution of R at position 100e with E (A14P and R100eE);
(7) substitution of A at position 55 with D, substitution of R at position 100e with E, and substitution of P at position 105 with Q (A55D, R100eE and P105Q);
(8) substitution of A at position 55 with D, substitution of W at position 100g with F, and substitution of D at position 102 with Y (A55D, W100gF and D102Y);
(9) substitution of A at position 55 with D, substitution of R at position 100e with E, and substitution of D at position 102 with Y (A55D, R100eE and D102Y);
(10) substitution of A at position 55 with D, and substitution of W at position 100g with F (A55D and W100gF);
(11) substitution of A at position 55 with D, substitution of R at position 100e with E, and substitution of W at position 100g with F (A55D, R100eE and W100gF);
(12) substitution of A at position 55 with D, and substitution of D at position 102 with Y (A55D and D102Y);
(13) substitution of L at position 48 with V, substitution of A at position 55 with D, and substitution of R at position 100e with E (L48V, A55D and R100eE);
(14) substitution of A at position 55 with D, substitution of R at position 100e with E, and substitution of P at position 105 with Q (A55D, R100eE and P105Q);
   or,
(15) substitution of A at position 55 with D, substitution of R at position 100e with E, substitution of W at position 100g with F, and substitution of P at position 105 with Q (A55D, R100eE, W100gF and P105Q);
wherein the positions are defined by the kabat numbering system.

In some embodiments, the single-domain antibody or antigen-binding fragment thereof comprises an amino acid sequence selected from the group consisting of:
(1) the amino acid sequence as set forth in positions 2-125 or positions 1-125 of SEQ ID NO: 2;
(2) the amino acid sequence as set forth in positions 2-125 or positions 1-125 of SEQ ID NO: 3;
(3) the amino acid sequence as set forth in positions 2-125 or positions 1-125 of SEQ ID NO: 4;
(4) the amino acid sequence as set forth in positions 2-125 or positions 1-125 of SEQ ID NO: 5;
(5) the amino acid sequence as set forth in positions 2-125 or positions 1-125 of SEQ ID NO: 6;
(6) the amino acid sequence as set forth in positions 2-125 or positions 1-125 of SEQ ID NO: 7;
(7) the amino acid sequence as set forth in positions 2-125 or positions 1-125 of SEQ ID NO: 8;
(8) the amino acid sequence as set forth in positions 2-125 or positions 1-125 of SEQ ID NO: 9;
(9) the amino acid sequence as set forth in positions 2-125 or positions 1-125 of SEQ ID NO: 10;
(10) the amino acid sequence as set forth in positions 2-125 or positions 1-125 of SEQ ID NO: 11;
(11) the amino acid sequence as set forth in positions 2-125 or positions 1-125 of SEQ ID NO: 12;
(12) the amino acid sequence as set forth in positions 2-125 or positions 1-125 of SEQ ID NO: 13;
(13) the amino acid sequence as set forth in positions 2-125 or positions 1-125 of SEQ ID NO: 15;
(14) the amino acid sequence as set forth in positions 2-125 or positions 1-125 of SEQ ID NO: 16;
(15) the amino acid sequence as set forth in positions 2-125 or positions 1-125 of SEQ ID NO: 17;
(16) the amino acid sequence as set forth in positions 2-125 or positions 1-125 of SEQ ID NO: 18;
(17) the amino acid sequence as set forth in positions 2-125 or positions 1-125 of SEQ ID NO: 19;
(18) the amino acid sequence as set forth in positions 2-125 or positions 1-125 of SEQ ID NO: 20;
(19) the amino acid sequence as set forth in positions 2-125 or positions 1-125 of SEQ ID NO: 21;
(20) the amino acid sequence as set forth in positions 2-125 or positions 1-125 of SEQ ID NO: 22;
(21) the amino acid sequence as set forth in positions 2-125 or positions 1-125 of SEQ ID NO: 23;
(22) the amino acid sequence as set forth in positions 2-125 or positions 1-125 of SEQ ID NO: 24;
(23) the amino acid sequence as set forth in positions 2-125 or positions 1-125 of SEQ ID NO: 25;
(24) the amino acid sequence as set forth in positions 2-125 or positions 1-125 of SEQ ID NO: 26;
(25) the amino acid sequence as set forth in positions 2-125 or positions 1-125 of SEQ ID NO: 27;
(26) the amino acid sequence as set forth in positions 2-125 or positions 1-125 of SEQ ID NO: 28;
(27) the amino acid sequence as set forth in positions 2-125 or positions 1-125 of SEQ ID NO: 29;
(28) the amino acid sequence as set forth in positions 2-125 or positions 1-125 of SEQ ID NO: 30;
(29) the amino acid sequence as set forth in positions 2-125 or positions 1-125 of SEQ ID NO: 31;
(30) the amino acid sequence as set forth in positions 2-125 or positions 1-125 of SEQ ID NO: 32;
(31) the amino acid sequence as set forth in positions 2-125 or positions 1-125 of SEQ ID NO: 33;
(32) the amino acid sequence as set forth in positions 2-125 or positions 1-125 of SEQ ID NO: 34;
(33) the amino acid sequence as set forth in positions 2-125 or positions 1-125 of SEQ ID NO: 35;
(34) the amino acid sequence as set forth in positions 2-125 or positions 1-125 of SEQ ID NO: 36.

In some embodiments, the GH is a human GH.

In some embodiments, the single-domain antibody or antigen-binding fragment thereof has superior thermostability and/or alkaline stability compared with the antibody as set forth in SEQ ID NO: 1 or SEQ ID NO: 37.

In a second aspect, the present application provides a single-domain antibody capable of specifically binding to a growth hormone (GH) or an antigen-binding fragment thereof, wherein the single-domain antibody or antigen-binding fragment thereof comprises CDR1, CDR2 and CDR3, and compared with the sequence as set forth in SEQ ID NO: 37, the single-domain antibody having one or more substitution mutations selected from the group consisting of:
(i) substitution of D at position 114 of SEQ ID NO: 37 with Y;
(ii) substitution of P at position 117 of SEQ ID NO: 37 with Q;
(iii) substitution of L at position 49 of SEQ ID NO: 37 with V;
(iv) substitution of L at position 12 of SEQ ID NO: 37 with K;
(v) substitution of W at position 112 of SEQ ID NO: 37 with F;
(vi) substitution of V at position 113 of SEQ ID NO: 37 with D;
(vii) substitution of P at position 29 of SEQ ID NO: 37 with T;
(viii) substitution of R at position 20 of SEQ ID NO: 37 with T;
(ix) substitution of T at position 58 of SEQ ID NO: 37 with K;
(x) substitution of R at position 110 of SEQ ID NO: 37 with E;
(xi) substitution of A at position 57 of SEQ ID NO: 37 with D;
(xii) substitution of N at position 78 of SEQ ID NO: 37 with S;
(xiii) substitution of A at position 15 of SEQ ID NO: 37 with P.

In some embodiments, compared with the sequence as set forth in SEQ ID NO: 37, the single-domain antibody has one or more substitution mutations selected from the group consisting of:
(a) substitution of D at position 114 of SEQ ID NO: 37 with Y;
(b) substitution of P at position 117 of SEQ ID NO: 37 with Q;
(c) substitution of L at position 49 of SEQ ID NO: 37 with V;
(d) substitution of W at position 112 of SEQ ID NO: 37 with F;
(e) substitution of P at position 29 of SEQ ID NO: 37 with T;
(f) substitution of R at position 20 of SEQ ID NO: 37 with T;
(g) substitution of R at position 110 of SEQ ID NO: 37 with E;
(h) substitution of A at position 57 of SEQ ID NO: 37 with D;
(i) substitution of A at position 15 of SEQ ID NO: 37 with P.

In some embodiments, compared with the sequence as set forth in SEQ ID NO: 37, the single-domain antibody has:
(1) substitution of A at position 57 of SEQ ID NO: 37 with D, and substitution of R at position 110 of SEQ ID NO: 37 with E;
(2) substitution of P at position 29 of SEQ ID NO: 37 with T, and substitution of A at position 57 of SEQ ID NO: 37 with D;
(3) substitution of A at position 15 of SEQ ID NO: 37 with P, and substitution of P at position 29 of SEQ ID NO: 37 with T;
(4) substitution of A at position 15 of SEQ ID NO: 37 with P, and substitution of P at position 117 of SEQ ID NO: 37 with Q;
(5) substitution of A at position 15 of SEQ ID NO: 37 with P, and substitution of R at position 20 of SEQ ID NO: 37 with T;
(6) substitution of A at position 15 of SEQ ID NO: 37 with P, and substitution of R at position 110 of SEQ ID NO: 37 with E;
(7) substitution of A at position 57 of SEQ ID NO: 37 with D, substitution of R at position 110 of SEQ ID NO: 37 with E, and substitution of P at position 117 of SEQ ID NO: 37 with Q;
(8) substitution of A at position 57 of SEQ ID NO: 37 with D, substitution of W at position 112 of SEQ ID NO: 37 with F, and substitution of D at position 114 of SEQ ID NO: 37 with Y;
(9) substitution of A at position 57 of SEQ ID NO: 37 with D, substitution of R at position 110 of SEQ ID NO: 37 with E, and substitution of D at position 114 of SEQ ID NO: 37 with Y;
(10) substitution of A at position 57 of SEQ ID NO: 37 with D, and substitution of W at position 112 of SEQ ID NO: 37 with F;
(11) substitution of A at position 57 of SEQ ID NO: 37 with D, substitution of R at position 110 of SEQ ID NO: 37 with E, and substitution of W at position 112 of SEQ ID NO: 37 with F;
(12) substitution of A at position 57 of SEQ ID NO: 37 with D, and substitution of D at position 114 of SEQ ID NO: 37 with Y;
(13) substitution of L at position 49 of SEQ ID NO: 37 with V, substitution of A at position 57 of SEQ ID NO: 37 with D, and substitution of R at position 110 of SEQ ID NO: 37 with E;
(14) substitution of A at position 57 of SEQ ID NO: 37 with D, substitution of R at position 110 of SEQ ID NO: 37 with E, and substitution of P at position 117 of SEQ ID NO: 37 with Q;
   or,
(15) substitution of A at position 57 of SEQ ID NO: 37 with D, substitution of R at position 110 of SEQ ID NO: 37 with E, substitution of W at position 112 of SEQ ID NO: 37 with F, and substitution of P at position 117 of SEQ ID NO: 37 with Q.

In some embodiments, the single-domain antibody or antigen-binding fragment thereof comprises an amino acid sequence selected from the group consisting of:
(1) the amino acid sequence as set forth in positions 2-125 or positions 1-125 of SEQ ID NO: 2;
(2) the amino acid sequence as set forth in positions 2-125 or positions 1-125 of SEQ ID NO: 3;
(3) the amino acid sequence as set forth in positions 2-125 or positions 1-125 of SEQ ID NO: 4;
(4) the amino acid sequence as set forth in positions 2-125 or positions 1-125 of SEQ ID NO: 5;
(5) the amino acid sequence as set forth in positions 2-125 or positions 1-125 of SEQ ID NO: 6;
(6) the amino acid sequence as set forth in positions 2-125 or positions 1-125 of SEQ ID NO: 7;
(7) the amino acid sequence as set forth in positions 2-125 or positions 1-125 of SEQ ID NO: 8;
(8) the amino acid sequence as set forth in positions 2-125 or positions 1-125 of SEQ ID NO: 9;
(9) the amino acid sequence as set forth in positions 2-125 or positions 1-125 of SEQ ID NO: 10;
(10) the amino acid sequence as set forth in positions 2-125 or positions 1-125 of SEQ ID NO: 11;
(11) the amino acid sequence as set forth in positions 2-125 or positions 1-125 of SEQ ID NO: 12;
(12) the amino acid sequence as set forth in positions 2-125 or positions 1-125 of SEQ ID NO: 13;
(13) the amino acid sequence as set forth in positions 2-125 or positions 1-125 of SEQ ID NO: 15;
(14) the amino acid sequence as set forth in positions 2-125 or positions 1-125 of SEQ ID NO: 16;
(15) the amino acid sequence as set forth in positions 2-125 or positions 1-125 of SEQ ID NO: 17;
(16) the amino acid sequence as set forth in positions 2-125 or positions 1-125 of SEQ ID NO: 18;
(17) the amino acid sequence as set forth in positions 2-125 or positions 1-125 of SEQ ID NO: 19;
(18) the amino acid sequence as set forth in positions 2-125 or positions 1-125 of SEQ ID NO: 20;
(19) the amino acid sequence as set forth in positions 2-125 or positions 1-125 of SEQ ID NO: 21;
(20) the amino acid sequence as set forth in positions 2-125 or positions 1-125 of SEQ ID NO: 22;
(21) the amino acid sequence as set forth in positions 2-125 or positions 1-125 of SEQ ID NO: 23;
(22) the amino acid sequence as set forth in positions 2-125 or positions 1-125 of SEQ ID NO: 24;
(23) the amino acid sequence as set forth in positions 2-125 or positions 1-125 of SEQ ID NO: 25;
(24) the amino acid sequence as set forth in positions 2-125 or positions 1-125 of SEQ ID NO: 26;
(25) the amino acid sequence as set forth in positions 2-125 or positions 1-125 of SEQ ID NO: 27;
(26) the amino acid sequence as set forth in positions 2-125 or positions 1-125 of SEQ ID NO: 28;
(27) the amino acid sequence as set forth in positions 2-125 or positions 1-125 of SEQ ID NO: 29;
(28) the amino acid sequence as set forth in positions 2-125 or positions 1-125 of SEQ ID NO: 30;
(29) the amino acid sequence as set forth in positions 2-125 or positions 1-125 of SEQ ID NO: 31;
(30) the amino acid sequence as set forth in positions 2-125 or positions 1-125 of SEQ ID NO: 32;
(31) the amino acid sequence as set forth in positions 2-125 or positions 1-125 of SEQ ID NO: 33;
(32) the amino acid sequence as set forth in positions 2-125 or positions 1-125 of SEQ ID NO: 34;
(33) the amino acid sequence as set forth in positions 2-125 or positions 1-125 of SEQ ID NO: 35;
(34) the amino acid sequence as set forth in positions 2-125 or positions 1-125 of SEQ ID NO: 36.

In some embodiments, the GH is a human GH.

In some embodiments, the single-domain antibody or antigen-binding fragment thereof has superior thermostability and/or alkaline stability compared with the antibody as set forth in SEQ ID NO: 1 or SEQ ID NO: 37.

In a third aspect, the present application provides a single-domain antibody capable of specifically binding to a growth hormone (GH) or an antigen-binding fragment thereof, wherein the single-domain antibody or antigen-binding fragment thereof comprises:
(1) CDR1 as set forth in SEQ ID NO: 42; CDR2 as set forth in SEQ ID NO: 43; and CDR3 as set forth in SEQ ID NO: 47;
(2) CDR1 as set forth in SEQ ID NO: 42; CDR2 as set forth in SEQ ID NO: 43; and CDR3 as set forth in SEQ ID NO: 48;
(3) CDR1 as set forth in SEQ ID NO: 42; CDR2 as set forth in SEQ ID NO: 43; and CDR3 as set forth in SEQ ID NO: 49;
(4) CDR1 as set forth in SEQ ID NO: 42; CDR2 as set forth in SEQ ID NO: 44; and CDR3 as set forth in SEQ ID NO: 46;
(5) CDR1 as set forth in SEQ ID NO: 42; CDR2 as set forth in SEQ ID NO: 43; and CDR3 as set forth in SEQ ID NO: 50;
(6) CDR1 as set forth in SEQ ID NO: 42; CDR2 as set forth in SEQ ID NO: 45; and CDR3 as set forth in SEQ ID NO: 46;
(7) CDR1 as set forth in SEQ ID NO: 42; CDR2 as set forth in SEQ ID NO: 45; and CDR3 as set forth in SEQ ID NO: 50;
(8) CDR1 as set forth in SEQ ID NO: 42; CDR2 as set forth in SEQ ID NO: 45; and CDR3 as set forth in SEQ ID NO: 51;
(9) CDR1 as set forth in SEQ ID NO: 42; CDR2 as set forth in SEQ ID NO: 45; and CDR3 as set forth in SEQ ID NO: 52;
(10) CDR1 as set forth in SEQ ID NO: 42; CDR2 as set forth in SEQ ID NO: 45; and CDR3 as set forth in SEQ ID NO: 48;
(11) CDR1 as set forth in SEQ ID NO: 42; CDR2 as set forth in SEQ ID NO: 45; and CDR3 as set forth in SEQ ID NO: 53;
   or,
(12) CDR1 as set forth in SEQ ID NO: 42; CDR2 as set forth in SEQ ID NO: 45; and CDR3 as set forth in SEQ ID NO: 47.

In some embodiments, the CDRs are as defined by the Kabat numbering system.

In some embodiments, the single-domain antibody or antigen-binding fragment thereof comprises an amino acid sequence selected from the group consisting of:
(i) the amino acid sequence as set forth in positions 2-125 or positions 1-125 of SEQ ID NO: 2;
(ii) the amino acid sequence as set forth in positions 2-125 or positions 1-125 of SEQ ID NO: 6;
(iii) the amino acid sequence as set forth in positions 2-125 or positions 1-125 of SEQ ID NO: 7;
(iv) the amino acid sequence as set forth in positions 2-125 or positions 1-125 of SEQ ID NO: 10;
(v) the amino acid sequence as set forth in positions 2-125 or positions 1-125 of SEQ ID NO: 11;
(vi) the amino acid sequence as set forth in positions 2-125 or positions 1-125 of SEQ ID NO: 12;
(vii) the amino acid sequence as set forth in positions 2-125 or positions 1-125 of SEQ ID NO: 16;
(viii) the amino acid sequence as set forth in positions 2-125 or positions 1-125 of SEQ ID NO: 17;
(ix) the amino acid sequence as set forth in positions 2-125 or positions 1-125 of SEQ ID NO: 23;
(x) the amino acid sequence as set forth in positions 2-125 or positions 1-125 of SEQ ID NO: 24;
(xi) the amino acid sequence as set forth in positions 2-125 or positions 1-125 of SEQ ID NO: 25;
(xii) the amino acid sequence as set forth in positions 2-125 or positions 1-125 of SEQ ID NO: 26;
(xiii) the amino acid sequence as set forth in positions 2-125 or positions 1-125 of SEQ ID NO: 27;
(xiv) the amino acid sequence as set forth in positions 2-125 or positions 1-125 of SEQ ID NO: 28;
(xv) the amino acid sequence as set forth in positions 2-125 or positions 1-125 of SEQ ID NO: 29;
(xvi) the amino acid sequence as set forth in positions 2-125 or positions 1-125 of SEQ ID NO: 30;
(xvii) the amino acid sequence as set forth in positions 2-125 or positions 1-125 of SEQ ID NO: 31;
(xviii) the amino acid sequence as set forth in positions 2-125 or positions 1-125 of SEQ ID NO: 32;
(xix) the amino acid sequence as set forth in positions 2-125 or positions 1-125 of SEQ ID NO: 33;
(xx) the amino acid sequence as set forth in positions 2-125 or positions 1-125 of SEQ ID NO: 34;
(xxi) the amino acid sequence as set forth in positions 2-125 or positions 1-125 of SEQ ID NO: 35;
(xxii) the amino acid sequence as set forth in positions 2-125 or positions 1-125 of SEQ ID NO: 36.

In some embodiments, the GH is a human GH.

In some embodiments, the single-domain antibody or antigen-binding fragment thereof has superior thermostability and/or alkaline stability compared with the antibody as set forth in SEQ ID NO: 1 or SEQ ID NO: 37.

In a fourth aspect, the present application provides a polypeptide construct capable of specifically binding to a growth hormone (GH), which comprises the single-domain antibody or antigen-binding fragment thereof of the first, second or third aspect, and an additional polypeptide.

In some embodiments, the polypeptide construct is a fusion protein.

In some embodiments, the additional polypeptide is optionally linked to the N-terminus or C-terminus of the single-domain antibody or antigen-binding fragment thereof via a linker.

In some embodiments, the additional polypeptide is selected from the group consisting of protein tags (e.g., His tags, such as HHHHHH or HHHHHHHH), protease recognition sequences (e.g., EK protease recognition sequences, such as DDDDK), peptide linkers (e.g., flexible peptide linkers; e.g., peptide linkers comprising one or more glycine (G) and/or serine (S)), or any combination thereof.

In some embodiments, the polypeptide construct comprises, in order from the N-terminus to the C-terminus: the single-domain antibody or antigen-binding fragment thereof of any one of claims 1 to 6, an EK protease recognition sequence (e.g., DDDDK), a flexible peptide linker (e.g., the peptide linker as set forth in SEQ ID NO: 40), and an optional His tag (e.g., HHHHHH or HHHHHHHH).

In some embodiments, the polypeptide construct comprises an amino acid sequence selected from the group consisting of:
(1) the amino acid sequence as set forth in SEQ ID NO: 2, or the amino acid sequence as set forth in positions 1-136, or positions 2-136, or positions 2-142 of SEQ ID NO: 2;
(2) the amino acid sequence as set forth in SEQ ID NO: 3, or the amino acid sequence as set forth in positions 1-136, or positions 2-136, or positions 2-142 of SEQ ID NO: 3;
(3) the amino acid sequence as set forth in SEQ ID NO: 4, or the amino acid sequence as set forth in positions 1-136, or positions 2-136, or positions 2-142 of SEQ ID NO: 4;
(4) the amino acid sequence as set forth in SEQ ID NO: 5, or the amino acid sequence as set forth in positions 1-136, or positions 2-136, or positions 2-142 of SEQ ID NO: 5;
(5) the amino acid sequence as set forth in SEQ ID NO: 6, or the amino acid sequence as set forth in positions 1-136, or positions 2-136, or positions 2-142 of SEQ ID NO: 6;
(6) the amino acid sequence as set forth in SEQ ID NO: 7, or the amino acid sequence as set forth in positions 1-136, or positions 2-136, or positions 2-142 of SEQ ID NO: 7;
(7) the amino acid sequence as set forth in SEQ ID NO: 8, or the amino acid sequence as set forth in positions 1-136, or positions 2-136, or positions 2-142 of SEQ ID NO: 8;
(8) the amino acid sequence as set forth in SEQ ID NO: 9, or the amino acid sequence as set forth in positions 1-136, or positions 2-136, or positions 2-142 of SEQ ID NO: 9;
(9) the amino acid sequence as set forth in SEQ ID NO: 10, or the amino acid sequence as set forth in positions 1-136, or positions 2-136, or positions 2-142 of SEQ ID NO: 10;
(10) the amino acid sequence as set forth in SEQ ID NO: 11, or the amino acid sequence as set forth in positions 1-136, or positions 2-136, or positions 2-142 of SEQ ID NO: 11;
(11) the amino acid sequence as set forth in SEQ ID NO: 12, or the amino acid sequence as set forth in positions 1-136, or positions 2-136, or positions 2-142 of SEQ ID NO: 12;
(12) the amino acid sequence as set forth in SEQ ID NO: 13, or the amino acid sequence as set forth in positions 1-136, or positions 2-136, or positions 2-142 of SEQ ID NO: 13;
(13) the amino acid sequence as set forth in SEQ ID NO: 15, or the amino acid sequence as set forth in positions 1-136, or positions 2-136, or positions 2-142 of SEQ ID NO: 15;
(14) the amino acid sequence as set forth in SEQ ID NO: 16, or the amino acid sequence as set forth in positions 1-136, or positions 2-136, or positions 2-142 of SEQ ID NO: 16;
(15) the amino acid sequence as set forth in SEQ ID NO: 17, or the amino acid sequence as set forth in positions 1-136, or positions 2-136, or positions 2-142 of SEQ ID NO: 17;
(16) the amino acid sequence as set forth in SEQ ID NO: 19, or the amino acid sequence as set forth in positions 1-136, or positions 2-136, or positions 2-142 of SEQ ID NO: 19;
(17) the amino acid sequence as set forth in SEQ ID NO: 20, or the amino acid sequence as set forth in positions 1-136, or positions 2-136, or positions 2-142 of SEQ ID NO: 20;
(18) the amino acid sequence as set forth in SEQ ID NO: 21, or the amino acid sequence as set forth in positions 1-136, or positions 2-136, or positions 2-142 of SEQ ID NO: 21;
(19) the amino acid sequence as set forth in SEQ ID NO: 23, or the amino acid sequence as set forth in positions 1-136, or positions 2-136, or positions 2-142 of SEQ ID NO: 23;
(20) the amino acid sequence as set forth in SEQ ID NO: 24, or the amino acid sequence as set forth in positions 1-136, or positions 2-136, or positions 2-142 of SEQ ID NO: 24;
(21) the amino acid sequence as set forth in SEQ ID NO: 25, or the amino acid sequence as set forth in positions 1-136, or positions 2-136, or positions 2-142 of SEQ ID NO: 25;
(22) the amino acid sequence as set forth in SEQ ID NO: 27, or the amino acid sequence as set forth in positions 1-136, or positions 2-136, or positions 2-142 of SEQ ID NO: 27;
(23) the amino acid sequence as set forth in SEQ ID NO: 29, or the amino acid sequence as set forth in positions 1-136, or positions 2-136, or positions 2-142 of SEQ ID NO: 29;
(24) the amino acid sequence as set forth in SEQ ID NO: 31, or the amino acid sequence as set forth in positions 1-136, or positions 2-136, or positions 2-142 of SEQ ID NO: 31;
(25) the amino acid sequence as set forth in SEQ ID NO: 34, or the amino acid sequence as set forth in positions 1-136, or positions 2-136, or positions 2-142 of SEQ ID NO: 34.

In some embodiments, the GH is a human GH.

In some embodiments, the construct has superior thermostability and/or alkaline stability compared with the antibody as set forth in SEQ ID NO: 1 or SEQ ID NO: 37.

In a fifth aspect, the present application provides a polypeptide construct capable of specifically binding to a growth hormone (GH), comprising a single-domain antibody capable of specifically binding to GH or an antigen-binding fragment thereof, and a polypeptide as set forth in SEQ ID NO: 41.

In some embodiments, the single-domain antibody capable of specifically binding to GH or antigen-binding fragment thereof is selected from: (i) the single-domain antibody or antigen-binding fragment thereof of the first, second or third aspect, and (ii) the single-domain antibody as set forth in SEQ ID NO: 37 or antigen-binding fragment thereof.

In some embodiments, the polypeptide construct is a fusion protein.

In some embodiments, the polypeptide construct comprises the polypeptide as set forth in SEQ ID NO: 41 at the C-terminus of the single-domain antibody capable of specifically binding to GH or antigen-binding fragment thereof.

In some embodiments, the polypeptide construct further comprises an EK protease recognition sequence (e.g., DDDDK) and/or a His tag (e.g., HHHHHH or HHHHHHHH).

In some embodiments, the polypeptide construct comprises, in order from the N-terminus to the C-terminus: the single-domain antibody capable of specifically binding to GH or antigen-binding fragment thereof, the EK protease recognition sequence, the polypeptide as set forth in SEQ ID NO: 41, and the optional His tag.

In some embodiments, the polypeptide construct comprises an amino acid sequence selected from the group consisting of:
(1) the amino acid sequence as set forth in SEQ ID NO: 14, or the amino acid sequence as set forth in positions 1-136, or positions 2-142, or positions 2-136 of SEQ ID NO: 14;
(2) the amino acid sequence as set forth in SEQ ID NO: 18, or the amino acid sequence as set forth in positions 1-136, or positions 2-142, or positions 2-136 of SEQ ID NO: 18;
(3) the amino acid sequence as set forth in SEQ ID NO: 22, or the amino acid sequence as set forth in positions 1-136, or positions 2-142, or positions 2-136 of SEQ ID NO: 22;
(4) the amino acid sequence as set forth in SEQ ID NO: 26, or the amino acid sequence as set forth in positions 1-136, or positions 2-142, or positions 2-136 of SEQ ID NO: 26;
(5) the amino acid sequence as set forth in SEQ ID NO: 28, or the amino acid sequence as set forth in positions 1-136, or positions 2-142, or positions 2-136 of SEQ ID NO: 28;
(6) the amino acid sequence as set forth in SEQ ID NO: 30, or the amino acid sequence as set forth in positions 1-136, or positions 2-142, or positions 2-136 of SEQ ID NO: 30;
(7) the amino acid sequence as set forth in SEQ ID NO: 32, or the amino acid sequence as set forth in positions 1-136, or positions 2-142, or positions 2-136 of SEQ ID NO: 32;
(8) the amino acid sequence as set forth in SEQ ID NO: 33, or the amino acid sequence as set forth in positions 1-136, or positions 2-142, or positions 2-136 of SEQ ID NO: 33;
(9) the amino acid sequence as set forth in SEQ ID NO: 35, or the amino acid sequence as set forth in positions 1-136, or positions 2-142, or positions 2-136 of SEQ ID NO: 35;
(10) the amino acid sequence as set forth in SEQ ID NO: 36, or the amino acid sequence as set forth in positions 1-136, or positions 2-142, or positions 2-136 of SEQ ID NO: 36.

In some embodiments, the GH is a human GH.

In some embodiments, the construct has superior thermostability and/or alkaline stability compared with the antibody as set forth in SEQ ID NO: 1 or SEQ ID NO: 37.

In a sixth aspect, the present application provides an isolated nucleic acid molecule encoding the single-domain antibody or antigen-binding fragment thereof of the first, second or third aspect, or the polypeptide construct of the fourth or fifth aspect.

In a seventh aspect, the present application provides a vector comprising the isolated nucleic acid molecule of the sixth aspect. In some embodiments, the vector is a cloning vector or an expression vector.

In an eighth aspect, the present application provides a host cell comprising the isolated nucleic acid molecule of the sixth aspect or the vector of the seventh aspect. Such host cells include, but are not limited to, prokaryotic cells such as bacterial cells (e.g., Escherichia coli cells), and eukaryotic cells such as fungal cells (e.g., yeast cells), insect cells, plant cells, and animal cells (e.g., mammalian cells, such as mouse cells and human cells). In some embodiments, the host cell is a microorganism.

The single-domain antibody or polypeptide construct of the present invention can be prepared by various methods known in the art, for example, by genetic engineering recombination technology. For example, DNA molecules encoding the single-domain antibody or construct of the present invention are obtained by chemical synthesis or PCR amplification. The resulting DNA molecules are inserted into an expression vector, followed by transformation/transfection of host cells. Subsequently, the transformed/transfected host cells are cultured under specific conditions to express the single-domain antibody or construct of the present invention.

The antigen-binding fragments of the present invention can be obtained by hydrolyzing intact nanobody molecules (see Morimoto et al., J. Biochem. Biophys. Methods 24:107-117 (1992) and Brennan et al., Science 229:81 (1985)). In addition, these antigen-binding fragments can also be produced directly from recombinant host cells (reviewed in Hudson, Curr. Opin. Immunol. 11: 548-557 (1999); Little et al., Immunol. Today, 21: 364-370 (2000)). Other technologies for preparing these antigen-binding fragments are well known to those of ordinary skill in the art.

In a ninth aspect, the present application provides a method for preparing the single-domain antibody or antigen-binding fragment thereof of the first, second or third aspect, or the polypeptide construct of the fourth or fifth aspect, comprising culturing the host cell of the eighth aspect under conditions permitting protein expression, and recovering the single-domain antibody or antigen-binding fragment thereof or the polypeptide construct from the culture of the cultured host cells.

In a tenth aspect, the present application provides a conjugate, comprising the single-domain antibody or antigen-binding fragment thereof of the first, second or third aspect, or the polypeptide construct of the fourth or fifth aspect, and a solid support linked to the single-domain antibody or antigen-binding fragment thereof or the polypeptide construct.

In some embodiments, the solid support is selected from: magnetic beads, agarose microspheres, polymeric matrices (e.g., dextran microspheres, polymethacrylate, polystyrene, polystyrene-divinylbenzene, poly(methyl propionate)), silica matrices (e.g., silica microspheres), graphitized carbon matrices, alumina matrices, zirconia matrices, and any combination thereof.

In some embodiments, the solid support is selected from porous materials, for example, a single porous material or a combination of multiple porous materials.

In an eleventh aspect, the present application provides a method for purifying GH, comprising using the conjugate of the tenth aspect.

In some embodiments, the method is a method for purifying GH by affinity chromatography.

In some embodiments, the method comprises the following steps:
(1) allowing the conjugate to bind to the GH in a first solvent to form a complex;
(2) dissociating the complex in a second solvent; and
(3) collecting the dissociated product containing the GH;
wherein the first solvent is a solvent suitable for forming a complex between the conjugate and the GH, and the second solvent is a solvent capable of dissociating the complex.

In some embodiments, the conductivity of the first solvent is 10-25 mS/cm (e.g., 15-19 mS/cm, 10-20 mS/cm, 15-25 mS/cm), and/or the conductivity of the second solvent is 0.3-5.0 mS/cm (e.g., 0.5-1.0 mS/cm, 0.5-3.0 mS/cm, 0.5-5.0 mS/cm, 1.0-3.0 mS/cm, 1.0-5.0 mS/cm).

In some embodiments, the pH of the first solvent is 6.0-8.5 (e.g., 7.3-7.7, 6.0-8.0, 6.5-7.7, 6.5-8.5, 7.0-7.7, 7.0-8.5), and/or the pH of the second solvent is 3.0-4.5 (e.g., 3.4-3.8, 3.0-3.8, 3.0-4.2, 3.4-4.2, 3.4-4.5).

In some embodiments, the GH is a human GH.

In a twelfth aspect, the present application provides use of the single-domain antibody or antigen-binding fragment thereof of the first, second or third aspect, or the polypeptide construct of the fourth or fifth aspect, or the conjugate of the tenth aspect, in the preparation of a reagent for GH purification.

In some embodiments, the GH is a human GH.

In a thirteenth aspect, the present application provides a conjugate, comprising the single-domain antibody or antigen-binding fragment thereof of the first, second or third aspect, or the polypeptide construct of the fourth or fifth aspect, and a detectable label linked to the single-domain antibody or antigen-binding fragment thereof or the polypeptide construct.

In some embodiments, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

In a fourteenth aspect, the present application provides a kit comprising the single-domain antibody or antigen-binding fragment thereof of the first, second or third aspect, or the polypeptide construct of the fourth or fifth aspect, or the conjugate of the thirteenth aspect.

In some embodiments, the kit comprises the conjugate of the thirteenth aspect.

In some embodiments, the kit comprises the single-domain antibody or antigen-binding fragment thereof of the first, second or third aspect, or the polypeptide construct of the fourth or fifth aspect, and a secondary antibody that specifically recognizes the single-domain antibody or antigen-binding fragment thereof or the polypeptide construct; optionally, the secondary antibody further comprises a detectable label, such as an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

In a fifteenth aspect, the present application provides a method for detecting the presence or level of GH in a sample, comprising using the single-domain antibody or antigen-binding fragment thereof of the first, second or third aspect, or the polypeptide construct of the fourth or fifth aspect, or the conjugate of the thirteenth aspect.

In some embodiments, the method is an immunological assay, such as Western blotting, enzyme immunoassay (e.g., ELISA), chemiluminescence immunoassay, fluorescence immunoassay or radioimmunoassay.

In some embodiments, the method comprises using the conjugate of the thirteenth aspect.

In some embodiments, the method comprises using the single-domain antibody or antigen-binding fragment thereof of the first, second or third aspect, or the polypeptide construct of the fourth or fifth aspect, and the method further comprises using a secondary antibody carrying a detectable label (e.g., an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin) to detect the single-domain antibody or antigen-binding fragment thereof or the polypeptide construct.

In some embodiments, the method comprises: (1) contacting the sample with the single-domain antibody or antigen-binding fragment thereof of the first, second or third aspect, or the construct of the fourth or fifth aspect, or the conjugate of the thirteenth aspect; (2) detecting the formation of an antigen-antibody immune complex or detecting the amount of the immune complex. The formation of the immune complex indicates the presence of GH.

In some embodiments, the GH is a human GH.

In some embodiments, the method is used for therapeutic purposes, diagnostic purposes, or non-therapeutic and non-diagnostic purposes.

In a sixteenth aspect, the present application further provides a method for diagnosing diseases associated with abnormal GH levels, comprising detecting the GH level in a sample from a subject using the method of the fifteenth aspect.

In some embodiments, a significantly increased or decreased GH level in the sample from the subject compared with a reference level (e.g., compared with a healthy control) indicates that the subject suffers from a disease associated with abnormally high or low GH levels (e.g., gigantism/dwarfism).

In some embodiments, the diseases associated with abnormal GH levels are selected from: endogenous GH deficiency, Noonan syndrome short stature, SHOX deficiency/short stature disorder, achondroplastic short stature, Turner syndrome, small for gestational age (SGA), and disease/stress-induced GH deficiency.

In some embodiments, the GH is a human GH.

In a seventeenth aspect, the present application provides use of the single-domain antibody or antigen-binding fragment thereof of the first, second or third aspect, or the polypeptide construct of the fourth or fifth aspect, or the conjugate of the thirteenth aspect, in the preparation of a detection reagent for detecting the presence or level of GH in a sample and/or diagnosing diseases associated with abnormal GH levels.

In some embodiments, the detection reagent is used to detect the presence or level of GH in a sample by the method of the fifteenth aspect.

In some embodiments, the diseases associated with abnormal GH levels are selected from: endogenous GH deficiency, Noonan syndrome short stature, SHOX deficiency/short stature disorder, achondroplastic short stature, Turner syndrome, small for gestational age (SGA), and disease/stress-induced GH deficiency.

In some embodiments, the sample is a body fluid sample from a subject (e.g., a mammal, preferably a human).

In some embodiments, the GH is a human GH.

### Definitions of terms

In the present invention, unless otherwise specified, the scientific and technical terms used herein have the meanings commonly understood by a person of skill in the art. Furthermore, the virology, biochemistry, and immunology laboratory operation procedures used herein are all conventional procedures widely used in the corresponding fields. Moreover, in order to better understand the present invention, definitions and explanations of relevant terms are provided below.

When the terms "for example", "e.g.", "such as", "including", "comprising", or variations thereof are used herein, these terms will not be considered as restrictive terms, but will be interpreted to mean "but not limited to" or "not limited to".

The terms "a", "an", "the", and similar referents in the context of describing the present invention (especially in the context of the following claims) should be construed to cover both the singular and the plural referents, unless otherwise indicated herein or clearly contradicted by context.

As used herein, the term "antibody" is used in the broadest sense, including but not limited to monoclonal antibodies (including human antibodies, humanized antibodies, or chimeric antibodies), polyclonal antibodies, multispecific antibodies (e.g., bispecific antibodies), and antibody fragments thereof, provided they exhibit the desired biological activity (e.g., antigen-binding activity).

As used herein, the term "antibody fragment" comprises a portion of an intact antibody, which partially or fully retains the antigen-binding activity of the antibody from which it is derived. The antibody fragment preferably comprises the antigen-binding region or variable region of an intact antibody. Non-limiting examples of antibody fragments include Fab, Fab', F(ab')2 and Fv fragments; diabodies; linear antibodies (Zapata et al., ProteinEng., 8(10): 1057-1062 (1995)); single-chain antibody molecules; and multispecific antibodies formed from antibody fragments.

As used herein, the term "single-domain antibody" has the meaning commonly understood by those skilled in the art, and refers to an antibody fragment composed of a single monomeric variable antibody domain (e.g., a single heavy chain variable region), which is generally derived from the variable region of a heavy chain antibody (e.g., a Camelid antibody or a shark antibody). Typically, a single-domain antibody consists of 4 framework regions and 3 complementarity-determining regions, with a structure of FR1-CDR1-FR2-CDR2-FR3-CDR3-FR4. Single-domain antibodies can be truncated at the N-terminus or C-terminus to contain only a portion of FR1 and/or FR4, or to lack one or both of those framework regions, as long as they essentially maintain antigen binding and specificity. A single-domain antibody is also referred to as a nanobody, and the two terms may be used interchangeably.

As used herein, the term "antigen-binding fragment" of a single-domain antibody refers to a polypeptide comprising a fragment of the single-domain antibody, which retains the ability to specifically bind to the same antigen bound by the single-domain antibody, and/or competes with the single-domain antibody for specific binding to the antigen, and is also referred to as an "antigen-binding portion". Generally, see Fundamental Immunology, Ch. 7 (Paul, W., ed., 2nd ed., Raven Press, N.Y. (1989), which is incorporated herein by reference in its entirety for all purposes. The antigen-binding fragments of the antibodies of the present invention can be produced by recombinant DNA technology or by enzymatic or chemical cleavage of the single-domain antibodies of the present invention. In some embodiments, the "antigen-binding fragment" of the single-domain antibody may be truncated at the N-terminus or C-terminus compared with the full-length single-domain antibody such that it contains only a portion of FR1 and/or FR4, or lacks one or both of those framework regions, provided that it substantially retains antigen binding and specificity.

Antigen-binding fragments of a single-domain antibody can be obtained from a given single-domain antibody (e.g., the nanobody provided by the present invention) using conventional techniques known to those skilled in the art (e.g., recombinant DNA technology, or enzymatic or chemical cleavage methods), and the antigen-binding fragments of the single-domain antibody can be screened for specificity in the same manner as that used for intact single-domain antibodies.

As used herein, unless the context clearly dictates otherwise, reference to the term "single-domain antibody" includes not only intact single-domain antibodies, but also antigen-binding fragments of the single-domain antibodies.

As used herein, the term "complementarity-determining region" or "CDR" refers to the amino acid residues in the variable region of an antibody that are responsible for antigen binding. A nanobody contains three CDRs, designated CDR1, CDR2 and CDR3. The precise boundaries of these CDRs can be defined according to various numbering systems known in the art, such as the definitions in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given nanobody, those skilled in the art can readily identify the CDRs defined by each numbering system. Furthermore, the corresponding relationship between different numbering systems is well known to a person of skill in the art (for example, see Lefranc et al., Dev. Comparat. Immunol. 27:55-77, 2003). As used herein, the CDRs of a nanobody are preferably determined by the Kabat numbering system.

As used herein, the term "framework region" or "FR" residues refer to those amino acid residues in the antibody variable region other than the CDR residues as defined above.

As used herein, the term "specific binding" refers to a non-random binding reaction between two molecules, such as the reaction between an antibody and a targeted antigen. The strength or affinity of a specific binding interaction can be expressed in terms of an equilibrium dissociation constant (K_{D}) for the interaction. In the present invention, the term "K_{D}" refers to a dissociation equilibrium constant for a specific antibody-antigen interaction, which is used for describing the binding affinity between an antibody and an antigen. The smaller the equilibrium dissociation constant, the tighter the antibody-antigen binding, and the higher the affinity between the antibody and the antigen.

The specific binding properties between two molecules can be determined by a method known in the art. One method involves measurement of the rates of formation and dissociation of antigen-binding sites/antigen complexes. Both the "association rate constant" (kₐ or kₒₙ) and the "dissociation rate constant" (k_{dis} or k_{off}) can be calculated by the concentration and the actual rates of association and dissociation (see Malmqvist M, Nature,1993, 361:186-187). The ratio of k_{dis}/kₒₙ is equal to the dissociation constant K_{D} (see Davies et al., Annual Rev Biochem, 1990; 59:439-473). The values of the K_{D}, kₒₙ and k_{dis} can be measured by any effective method. In some embodiments, the dissociation constant can be measured in Biacore by surface plasmon resonance (SPR). In addition, bioluminescence interferometry or Kinexa can be used for measuring the dissociation constant.

As used herein, the detectable label of the present invention may be any substance detectable by fluorescent, spectroscopic, photochemical, biochemical, immunological, electrical, optical or chemical means. Such labels are well known in the art, and examples thereof include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluoresceins, tetramethylrhodamine isothiocyanate (TRITC), phycoerythrin (PE), Texas Red, rhodamine, quantum dots or cyanine dye derivatives (e.g., Cy7 and Alexa 750)), luminescent substances (e.g., chemiluminescent substances, such as acridinium ester compounds, luminol and derivatives thereof, and ruthenium derivatives such as terpyridine ruthenium), magnetic beads (e.g., Dynabeads^{®}), colorimetric labels such as colloidal gold or colored glass or plastic (e.g., polystyrene, polypropylene, latex, etc.) beads, and biotins for binding to avidins (e.g., streptavidin) modified with the above labels.

As used herein, the term "vector" refers to a nucleic acid delivery vehicle into which a polynucleotide can be inserted. When a vector can express the protein encoded by the inserted polynucleotide, the vector is called an expression vector. The vector can be introduced into the host cell by transformation, transduction or transfection, so that the genetic material elements carried by the vector are expressed in the host cell. Vectors are well known to those skilled in the art, and include but are not limited to: plasmids; bacteriophagemids; cosmids; artificial chromosomes, such as yeast artificial chromosomes (YACs), bacterial artificial chromosomes (BACs), or P1-derived artificial chromosomes (PACs); and bacteriophages such as λ bacteriophages or M13 bacteriophages and animal viruses. Animal viruses that can be used as vectors include, but are not limited to, retroviruses (including lentiviruses), adenoviruses, adeno-associated viruses, herpes viruses (e.g., herpes simplex viruses), poxviruses, baculoviruses, papillomaviruses, and papovaviruses (e.g., SV40). A vector may comprise a variety of elements for controlling expression, including, but not limited to, promoter sequences, transcription initiation sequences, enhancer sequences, selection elements, and reporter genes. In addition, the vector may also comprise a replication origin.

As used herein, the term "host cell" refers to a cell that can be used for introducing a vector, including but not limited to prokaryotic cells such as Escherichia coli or Bacillus subtilis, fungal cells such as yeast cells or Aspergillus, insect cells such as S2 Drosophila cells or Sf9, or animal cells such as fibroblasts, CHO cells, COS cells, NSO cells, HeLa cells, BHK cells, HEK 293 cells or human cells.

Twenty conventional amino acids mentioned herein are written in accordance with conventional usage. See, e.g. Immunology-A Synthesis (2nd Edition, E. S. Golub and D. R. Gren, Eds., Sinauer Associates , Sunderland, Mass. (1991)), which is incorporated herein by reference. In the present invention, the terms "polypeptide" and "protein" have the same meaning and are used interchangeably. Moreover, in the present invention, amino acids are generally represented by single-letter and three-letter abbreviations well known in the art. For example, alanine can be represented by A or Ala.

### Beneficial effects of the invention

The series of single-domain antibodies against growth hormones (e.g., human growth hormones) provided herein possess high binding activity to growth hormones (e.g., human growth hormones), while exhibiting significantly excellent thermal tolerance and alkali resistance (e.g., being tolerant to treatment with 0.3 M or 0.5M alkali for 24h, and/or having a 5-10°C higher Tₘ compared with the parental antibody). Advantageously, they can be applied to the affinity purification of growth hormones (e.g., human growth hormones). For example, compared with existing anti-GH antibodies, the immunoaffinity media prepared from the single-domain antibodies of the present application are easier to clean, have an extended service life, and feature a prolonged shelf life for storage at room temperature. In addition, the single-domain antibody features low molecular weight and ease of production.

The embodiments of the present invention will be described in detail below in conjunction with the accompanying drawings and examples, but a person of skill in the art will understand that the following accompanying drawings and examples are only used for illustrating the present invention rather than limiting the scope of the present invention. Various purposes and advantages of the present invention will become apparent to a person of skill in the art according to the accompanying drawings and the following detailed descriptions of the preferred embodiments.

### BRIEF DESCRIPTION OF THE DRAWINGS

Fig. 1: ELISA detection results of 45 single-point mutants after treatment with 0.3 M NaOH for 24h.
Fig. 2: ELISA detection results of 14 single-point mutants after treatment with 0.5M NaOH for 24h.
Fig. 3: SDS-PAGE detection results of 10 single-point mutants after treatment with 0.5M alkali.
Fig. 4: Tm value detection results of 10 single-point mutants.
Fig. 5: SDS-PAGE electrophoresis results of alkali resistance of 40 multi-point mutants (Fig. 5A) and the corresponding statistical results (Fig. 5B).
Fig. 6: Absolute EC₅₀ values of 21 multi-point mutants.
Fig. 7: Tm value detection results of 21 multi-point mutants.
Fig. 8: Electrophoresis detection results of preferred molecules after alkali treatment.
Fig. 9: Alkali resistance test results of conjugated media of 6 preferred molecules.

### Sequence information

The description of the sequences involved in the present application is provided in the following table.

**Table 1: Sequence information**

| Designa tion | Mutation site (Based on SEQ ID NO: 1) | Mutation site (Based on Kabat numbering system) | Sequence information (Mutation sites are underlined) | SEQ ID NO: |
|---|---|---|---|---|
| WT (with tag) | - | - | MQVQLVESGGGLAQAGGSLRLSCAVSGMPEFARAMG WFRQAPGKERELLAAIEGIGATTYYADSVKGRFTISRDD AANTVLLQMNSLKPDDTAVYYCAAAFSVTIPTRARHW VDWGPGTLVTVSSDDDDKSGGGGSHHHHHH | 1 |
| M8 | D114Y | D102Y | MQVQLVESGGGLAQAGGSLRLSCAVSGMPEFARAMG WFRQAPGKERELLAAIEGIGATTYYADSVKGRFTISRDD AANTVLLQMNSLKPDDTAVYYCAAAFSVTIPTRARHW VYWGPGTLVTVSSDDDDKSGGGGSHHHHHH | 2 |
| M11 | P117Q | P105Q | MQVQLVESGGGLAQAGGSLRLSCAVSGMPEFARAMG WFRQAPGKERELLAAIEGIGATTYYADSVKGRFTISRDD AANTVLLQMNSLKPDDTAVYYCAAAFSVTIPTRARHW VDWGQGTLVTVSSDDDDKSGGGGSHHHHHH | 3 |
| M20 | L49V | L48V | MQVQLVESGGGLAQAGGSLRLSCAVSGMPEFARAMG WFRQAPGKERELVAAIEGIGATTYYADSVKGRFTISRD DAANTVLLQMNSLKPDDTAVYYCAAAFSVTIPTRARH WVDWGPGTLVTVSSDDDDKSGGGGSHHHHHH | 4 |
| M22 | L12K | L11K | MQVQLVESGGGKAQAGGSLRLSCAVSGMPEFARAMG WFRQAPGKERELLAAIEGIGATTYYADSVKGRFTISRDD AANTVLLQMNSLKPDDTAVYYCAAAFSVTIPTRARHW VDWGPGTLVTVSSDDDDKSGGGGSHHHHHH | 5 |
| M24 | W112F | W100gF | MQVQLVESGGGLAQAGGSLRLSCAVSGMPEFARAMG WFRQAPGKERELLAAIEGIGATTYYADSVKGRFTISRDD ΛΛNTVLLQMNSLKPDDTΛVYYCΛΛΛFSVTIPTRΛRHFV DWGPGTLVTVSSDDDDKSGGGGSHHHHHH | 6 |
| M25 | V113D | V101D | MQVQLVESGGGLAQAGGSLRLSCAVSGMPEFARAMG WFRQAPGKERELLAAIEGIGATTYYADSVKGRFTISRDD AANTVLLQMNSLKPDDTAVYYCAAAFSVTIPTRARHW DDWGPGTLVTVSSDDDDKSGGGGSHHHHHH | 7 |
| M27 | P29T | P28T | MQVQLVESGGGLAQAGGSLRLSCAVSGMTEFARAMG WFRQAPGKERELLAAIEGIGATTYYADSVKGRFTISRDD AANTVLLQMNSLKPDDTAVYYCAAAFSVTIPTRARHW VDWGPGTLVTVSSDDDDKSGGGGSHHHHHH | 8 |
| M28 | R20T | R19T | MQVQLVESGGGLAQAGGSLTLSCAVSGMPEFARAMG WFRQAPGKERELLAAIEGIGATTYYADSVKGRFTISRDD AANTVLLQMNSLKPDDTAVYYCAAAFSVTIPTRARHW VDWGPGTLVTVSSDDDDKSGGGGSHHHHHH | 9 |
| M29 | T58K | T56K | MQVQLVESGGGLAQAGGSLRLSCAVSGMPEFARAMG WFRQAPGKERELLAAIEGIGAKTYYADSVKGRFTISRD DAANTVLLQMNSLKPDDTAVYYCAAAFSVTIPTRARH WVDWGPGTLVTVSSDDDDKSGGGGSHHHHHH | 10 |
| M31 | R110E | R100eE | MQVQLVESGGGLAQAGGSLRLSCAVSGMPEFARAMG WFRQAPGKERELLAAIEGIGATTYYADSVKGRFTISRDD AANTVLLQMNSLKPDDTAVYYCAAAFSVTIPTRAEHW VDWGPGTLVTVSSDDDDKSGGGGSHHHHHH | 11 |
| M33 | A57D | A55D | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRARHW VDWGPGTLVTVSSDDDDKSGGGGSHHHHHH | 12 |
| M34 | N78S | N76S | MQVQLVESGGGLAQAGGSLRLSCAVSGMPEFARAMG WFRQAPGKERELLAAIEGIGATTYYADSVKGRFTISRDD AASTVLLQMNSLKPDDTAVYYCAAAFSVTIPTRARHW VDWGPGTLVTVSSDDDDKSGGGGSHHHHHH | 13 |
| M45 | G134R | - | MQVQLVESGGGLAQAGGSLRLSCAVSGMPEFARAMG WFRQAPGKERELLAAIEGIGATTYYADSVKGRFTISRDD AANTVLLQMNSLKPDDTAVYYCAAAFSVTIPTRARHW VDWGPGTLVTVSSDDDDKSGGRGSHHHHHH | 14 |
| M50 | A15P | A14P | MQVQLVESGGGLAQPGGSLRLSCAVSGMPEFARAMG WFRQAPGKERELLAAIEGIGATTYYADSVKGRFTISRDD AANTVLLQMNSLKPDDTAVYYCAAAFSVTIPTRARHW VDWGPGTLVTVSSDDDDKSGGGGSHHHHHH | 15 |
| M53 | A57D; R110E | A55D; R100eE | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRAEHW VDWGPGTLVTVSSDDDDKSGGGGSHHHHHH | 16 |
| M59 | A57D; P29T | P28T; A55D | MQVQLVESGGGLAQAGGSLRLSCAVSGMTEFARAMG WFRQΛPGKERELLΛΛIEGIGDTTYYΛDSVKGRFTISRDD AANTVLLQMNSLKPDDTAVYYCAAAFSVTIPTRARHW VDWGPGTLVTVSSDDDDKSGGGGSHHHHHH | 17 |
| M64 | A15P; G134R | A14P | MQVQLVESGGGLAQPGGSLRLSCAVSGMPEFARAMG WFRQAPGKERELLAAIEGIGATTYYADSVKGRFTISRDD AANTVLLQMNSLKPDDTAVYYCAAAFSVTIPTRARHW VDWGPGTLVTVSSDDDDKSGGRGSHHHHHH | 18 |
| M66 | P29T; A15P | A14P; P28T | MQVQLVESGGGLAQPGGSLRLSCAVSGMTEFARAMG WFRQAPGKERELLAAIEGIGATTYYADSVKGRFTISRDD ΛΛNTVLLQMNSLKPDDTΛVYYCΛΛΛFSVTIPTRΛRHW VDWGPGTLVTVSSDDDDKSGGGGSHHHHHH | 19 |
| M67 | A15P; P117Q | A14P; P105Q | MQVQLVESGGGLAQPGGSLRLSCAVSGMPEFARAMG WFRQAPGKERELLAAIEGIGATTYYADSVKGRFTISRDD AANTVLLQMNSLKPDDTAVYYCAAAFSVTIPTRARHW VDWGQGTLVTVSSDDDDKSGGGGSHHHHHH | 20 |
| M68 | A15P; R20T | A14P; R19T | MQVQLVESGGGLAQPGGSLTLSCAVSGMPEFARAMG WFRQAPGKERELLAAIEGIGATTYYADSVKGRFTISRDD AANTVLLQMNSLKPDDTAVYYCAAAFSVTIPTRARHW VDWGPGTLVTVSSDDDDKSGGGGSHHHHHH | 21 |
| M69 | P29T; G134R | P28T | MQVQLVESGGGLAQAGGSLRLSCAVSGMTEFARAMG WFRQAPGKERELLAAIEGIGATTYYADSVKGRFTISRDD AANTVLLQMNSLKPDDTAVYYCAAAFSVTIPTRARHW VDWGPGTLVTVSSDDDDKSGGRGSHHHHHH | 22 |
| M70 | A15P; R110E | A14P; R100eE | MQVQLVESGGGLAQPGGSLRLSCAVSGMPEFARAMG WFRQAPGKERELLAAIEGIGATTYYADSVKGRFTISRDD ΛΛNTVLLQMNSLKPDDTΛVYYCΛΛΛFSVTIPTRΛEHW VDWGPGTLVTVSSDDDDKSGGGGSHHHHHH | 23 |
| M71 | A57D; P117Q; R110E | A55D; R100eE; P105Q | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRAEHW VDWGQGTLVTVSSDDDDKSGGGGSHHHHHH | 24 |
| M72 | A57D; D114Y; W112F | A55D; W100gF; D102Y | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRARHFV YWGPGTLVTVSSDDDDKSGGGGSHHHHHH | 25 |
| M74 | A57D; G134R; R110E | A55D; R100eE | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRAEHW VDWGPGTLVTVSSDDDDKSGGRGSHHHHHH | 26 |
| M75 | A57D; R110E; D114Y | A55D; R100eE; D102Y | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRAEHW VYWGPGTLVTVSSDDDDKSGGGGSHHHHHH | 27 |
| M76 | A57D; G134R; W112F | A55D; W100gF | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRARHFV DWGPGTLVTVSSDDDDKSGGRGSHHHHHH | 28 |
| M77 | A57D; R110E; W112F | A55D; R100eE; W100gF | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRAEHFV DWGPGTLVTVSSDDDDKSGGGGSHHHHHH | 29 |
| M78 | A57D; G134R; D114Y | A55D; D102Y | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRARHW VYWGPGTLVTVSSDDDDKSGGRGSHHHHHH | 30 |
| M79 | A57D; R110E; L49V | L48V; A55D; R100eE | MQVQLVESGGGLAQAGGSLRLSCAVSGMPEFARAMG WFRQΛPGKERELVΛΛIEGIGDTTYYΛDSVKGRFTISRD DAANTVLLQMNSLKPDDTAVYYCAAAFSVTIPTRAEH WVDWGPGTLVTVSSDDDDKSGGGGSHHHHHH | 31 |
| M81 | A57D; G134R; P117Q; R110E | A55D; R100eE; P105Q | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRAEHW VDWGQGTLVTVSSDDDDKSGGRGSHHHHHH | 32 |
| M82 | A57D; G134R; R110E; D114Y | A55D; R100eE; D102Y | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRAEHW VYWGPGTLVTVSSDDDDKSGGRGSHHHHHH | 33 |
| M83 | A57D; P117Q; R110E; W112F | A55D; R100eE; W100gF; P105Q | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRAEHFV DWGQGTLVTVSSDDDDKSGGGGSHHHHHH | 34 |
| M88 | A57D; G134R; R110E; L49V | L48V; A55D; R100eE | MQVQLVESGGGLAQAGGSLRLSCAVSGMPEFARAMG WFRQAPGKERELVAAIEGIGDTTYYADSVKGRFTISRD DAANTVLLQMNSLKPDDTAVYYCAAAFSVTIPTRAEH WVDWGPGTLVTVSSDDDDKSGGRGSHHHHHH | 35 |
| M89 | A57D; G134R; R110E; W112F | A55D; R100eE; W100gF | MQVQLVESGGGLAQAGGSLRLSCAVSGMPEFARAMG WFRQAPGKERELLAAIEGIGDTTYYADSVKGRFTISRDD AANTVLLQMNSLKPDDTAVYYCAAAFSVTIPTRAEHFV DWGPGTLVTVSSDDDDKSGGRGSHHHHHH | 36 |
| WT | - | - | MQVQLVESGGGLAQAGGSLRLSCAVSGMPEFARAMG WFRQAPGKERELLAAIEGIGATTYYADSVKGRFTISRDD AANTVLLQMNSLKPDDTAVYYCAAAFSVTIPTRARHW VDWGPGTLVTVSS | 37 |
| EK protease recognit ion sequenc e | - | - | DDDDK | 38 |
| His tag | - | - | HHHHHH | 39 |
| Linker-1 | - | - | SGGGGS | 40 |
| Linker-2 | - | - | SGGRGS | 41 |
| CDR1 | - | - | ARAMG | 42 |
| CDR2 | - | - | AIEGIGATTYYADSVKG | 43 |
| CDR2 | - | - | AIEGIGAKTYYADSVKG | 44 |
| CDR2 | - | - | AIEGIGDTTYYADSVKG | 45 |
| CDR3 | - | - | AFSVTIPTRARHWVD | 46 |
| CDR3 | - | - | AFSVTIPTRARHWVY | 47 |
| CDR3 | - | - | AFSVTIPTRARHFVD | 48 |
| CDR3 | - | - | AFSVTIPTRARHWDD | 49 |
| CDR3 | - | - | AFSVTIPTRAEHWVD | 50 |
| CDR3 | - | - | AFSVTIPTRARHFVY | 51 |
| CDR3 | - | - | AFSVTIPTRAEHWVY | 52 |
| CDR3 | - | - | AFSVTIPTRAEHFVD | 53 |

### DETAILED DESCRIPTION OF THE INVENTION

The present invention will be described with reference to the following examples which are intended to illustrate the present invention but not to limit the present invention.

Unless otherwise specified, the molecular biology experimental methods and immunoassays used in the present invention are basically performed based on the methods described in J. Sambrook et al., Molecular Cloning: Laboratory Manual, 2nd edition, Cold Spring Harbor Laboratory Press, 1989, and F. M. Ausubel et al., Compiled Molecular Biology Experiment Guide, 3rd edition, John Wiley & Sons, Inc., 1995. A person of skill in the art will appreciate that the Examples describe the present invention by way of examples and are not intended to limit the scope of protection claimed for the present invention.

### Example 1: Sample preparation

### 1.1 Protein design and preparation

Site-directed mutagenesis was designed for the anti-human growth hormone single-domain antibody screened by Changchun GeneScience Pharmaceutical Co., Ltd. (the sequence of which is as set forth in SEQ ID NO: 37, see Patent Application No. PCT/CN2023/096259) to optimize its heat resistance and alkali resistance. In addition, to facilitate protein identification and/or purification, a His-tag (SEQ ID NO: 39) and an EK protease recognition sequence (SEQ ID NO: 38) were fused to the C-terminus of the antibody. The EK protease recognition sequence and the His-tag were connected via a linker (e.g., the linker as set forth in SEQ ID NO: 40 or 41).

After protein design and single-site or multi-site mutagenesis, the sequences were artificially synthesized and constructed, and then used to transform Escherichia coli host cells for the expression of target proteins. After fermentation and culture of the host strains, the cells were disrupted by ultrasonication, and the target protein solution was obtained by nickel column affinity purification. The protein concentrate was further prepared by ultrafiltration centrifugation using ultrafiltration tubes. The protein was subjected to disulfide bond renaturation with urea, oxidizing and reducing agents, followed by secondary purification. The resulting purified protein was used as the modified target protein for analysis.

### 1.2 Experimental design

The target protein was subjected to DSF differential scanning to determine Tₘ; the protein was subjected to alkali treatment, followed by pH neutralization of the treated protein. The protein underwent Elisa for detection of binding affinity EC₅₀ and electrophoretic purity analysis before and after treatment. Under the current fermentation and preparation conditions, the data of modified proteins before and after treatment were compared with those of the unmodified GH single-domain antibody molecule (amino acid sequence as set forth in SEQ ID NO: 1). An increased Tₘ after optimization indicates improved protein thermostability; reduced degraded bands in electrophoresis results after alkali treatment suggest enhanced alkali resistance; equivalent or decreased EC₅₀ represents equivalent or enhanced affinity. Superior iterative GH single-domain antibody molecules were screened according to the heat resistance and alkali resistance of the optimized proteins.

### Example 2: Alkali resistance evaluation of first-round designed proteins (single-point mutants):

The untreated samples were subjected to Elisa binding affinity detection; meanwhile, 45 single-point mutants were treated with alkali to evaluate their alkali resistance. In the alkali treatment process, 0.3 M sodium hydroxide (NaOH) was firstly used, and the changes in binding affinity of the mutants were detected by Elisa. Then the mutants with improved alkali resistance were further treated with 0.5M sodium hydroxide and tested again. Meanwhile, the proportion of electrophoretic degradation bands of the preferred proteins after 0.5M treatment was investigated.

### 2.1 Antibody Elisa affinity detection

The rhGH protein (see UniProt: P01241) was diluted to 5 ug/ml, coated at 50 ul/well overnight, and then blocked. Samples were diluted with PBS to 40 ug/ml, and 12 concentration points were prepared via 2-fold serial dilution. Each serially diluted sample was added to the ELISA at 50 ul/well and incubated at room temperature for 1 h. The Anti-6*His HRP secondary antibody was diluted at 1:5000 with PBS, added at 50 ul/well, and incubated at room temperature for 1 h. Color development was performed, and the reaction was terminated by adding 2 M sulfuric acid. Measurement was conducted at OD450 using a microplate reader.

Results processing: ELISA curves for each sample were plotted based on OD450, and the alkali stability of the single-domain antibodies was evaluated by comparing the changes in ELISA curves and EC₅₀.

### 2.2 Antibody Elisa alkali resistance assay

Sample alkali treatment: The samples were diluted to 2 mg/ml with PBS; the diluted sample solutions were mixed with 0.6 M NaOH / 1 M NaOH solution at 1:1 (V/V), gently blended, and placed in a refrigerator at 2-8°C, samples were collected at the 24h treatment time point, and the treatment was terminated;

Termination of sample treatment: HCl solution was added at 1/2 the volume of the collected sample, mixed gently to adjust the solution to neutral, and the sample was stored at -80°C for later use. The detection method is the same as that for Elisa affinity assay.

### 2.3 Antibody SDS-PAGE detection

SDS-PAGE detection was performed on the alkali-treated samples: The samples were diluted with ultrapure water, 5X protein buffer was added at a ratio of 4:1, and the samples were heated at 95°C for 10 min. Using 15% SDS-PAGE gel, electrophoresis was performed at a constant voltage of 120V for 65 min, followed by staining and destaining. A fixed total protein loading amount of 4 µg was applied to detect both the control proteins and the proteins after 24h of alkali treatment. ImageJ and Imagelab software were used for relative quantitative analysis of small molecule bands of the 24h-treated proteins to determine protein degradation, so as to intuitively evaluate the alkali resistance of the proteins.

### 2.4 Results

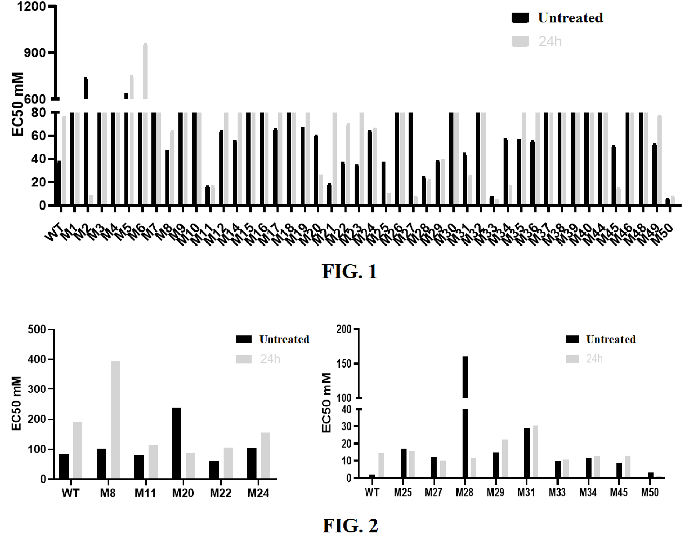

Fig. 1 shows the ELISA detection results of 45 single-point mutants treated with 0.3M NaOH for 24h.

Two types of single-point mutants were screened under the following criteria: (1) the affinity was equal to or higher than that of the WT without treatment, and the affinity remained equal to or higher than that of WT after alkali treatment; (2) the affinity was lower than that of the untreated WT, while the affinity after alkali treatment was equal to or higher than that of the WT. Based on this screening criteria, a total of 14 excellent single-point mutants were selected from the 45 mutants, namely M8, M11, M20, M22, M24, M25, M27, M28, M29, M31, M33, M34, M45 and M50.

Fig. 2 shows the ELISA detection results of further alkali treatment with 0.5M sodium hydroxide on the above 14 single-point mutants.

The results indicate that 10 of these single-point mutants exhibit superior alkali resistance under 0.5M, including M11, M20, M22, M24, M27, M28, M33, M34, M45 and M50. Their EC₅₀ values are approximately 50% higher than that of the WT after treatment with 0.5M alkali. Based on the above results, it is preliminarily confirmed that 10 out of the 45 initially expressed protein mutants maintain excellent stability upon 0.5M NaOH treatment.

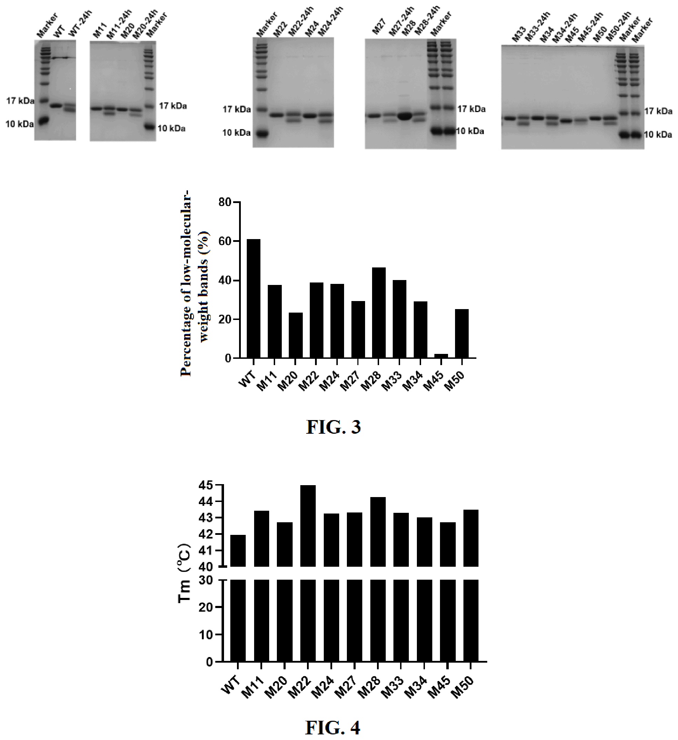

Fig. 3 shows the results of SDS-PAGE detection and calculation of the above 10 mutants. The calculation results demonstrate that the mutant proteins possess favorable stability. The proportion of small-molecule fragmented bands in the total protein is reduced by 30-50% compared with the WT.

### Example 3: Heat resistance evaluation of first-round designed proteins (single-point mutants):

Existing literature reports show that protein stability is consistent; that is, the improvement of one specific stability will enhance the stability under other conditions to varying degrees. Therefore, the Tm values of some alkali-resistant proteins were also tested to verify the improvement of their stability.

### 3.1 Detection of antibody heat resistance

Sample preparation: An appropriate amount of each sample was taken, diluted with buffer to the same concentration, mixed evenly and set aside for later use.

Experimental procedure for Tm detection by DSF method: The above samples were tested using a DSF instrument. The test proteins were diluted to a concentration of 0.1 mg/mL with PB buffer; 100×Spyro Orange dye was diluted to 20× with PB buffer; the protein and dye were mixed at a ratio of 19:1 (total volume 20 µL), with three parallel replicates for each sample, and PB buffer was used as the blank control. After shaking and mixing thoroughly, the samples were detected by a qPCR instrument (Jena qTower3, with excitation filter at 470 nm, and emission filter at 625 nm). The temperature was increased from 25°C to 95°C at a rate of 0.5°C/min, with equilibration for 5 s at each temperature. Finally, the Tm value was calculated by fitting the Boltzmann equation.

### 3.2 Results

The heat resistance detection results are shown in Fig. 4. The Tm values of the above 10 single-point mutants (M11, M20, M22, M24, M27, M28, M33, M34, M45, M50) were increased to varying degrees compared with the WT. That is, the heat resistance of the above mutants was also improved to varying degrees.

### Example 4: Investigation of alkali resistance of second-round designed proteins (multi-point mutants)

For multi-point mutants, the reduction in degraded bands after alkali treatment while maintaining high affinity was adopted as the main criterion for alkali resistance screening. The Elisa affinity detection method, alkali resistance detection method and SDS-PAGE detection method are the same as those described in Example 2.

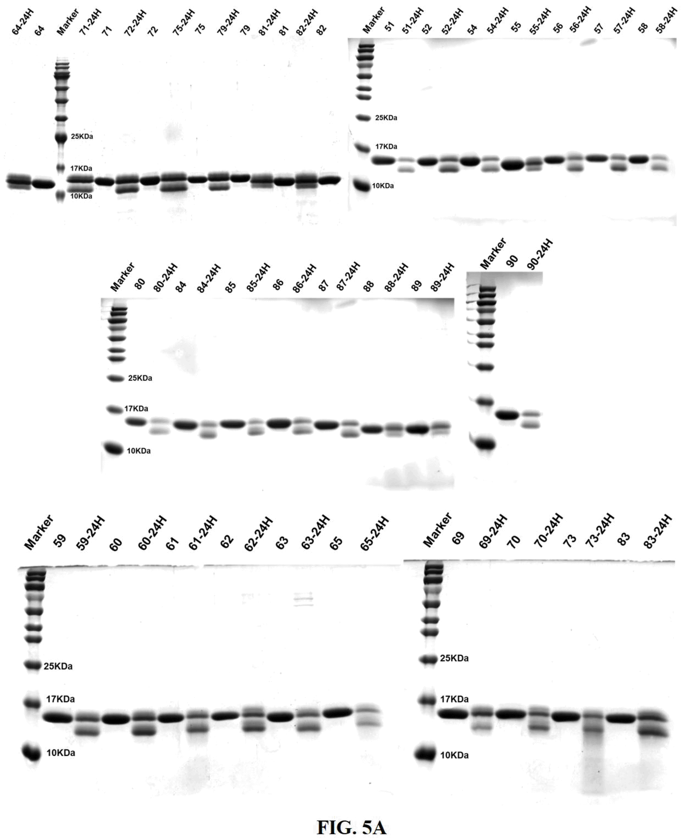

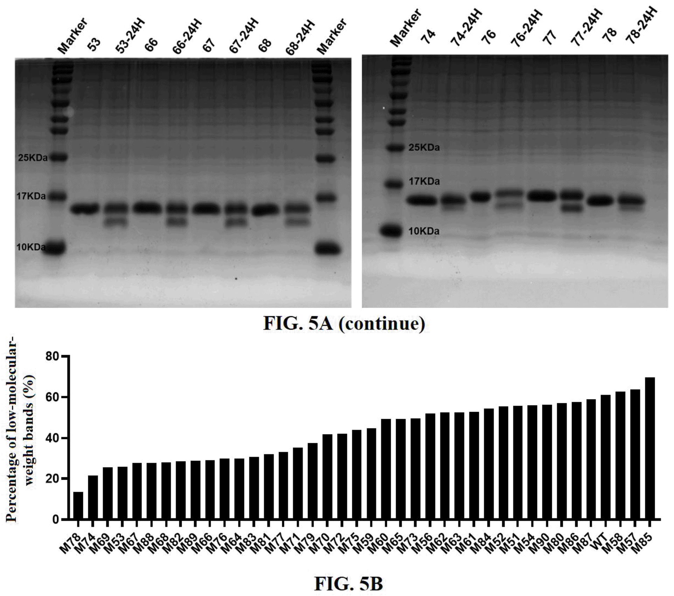

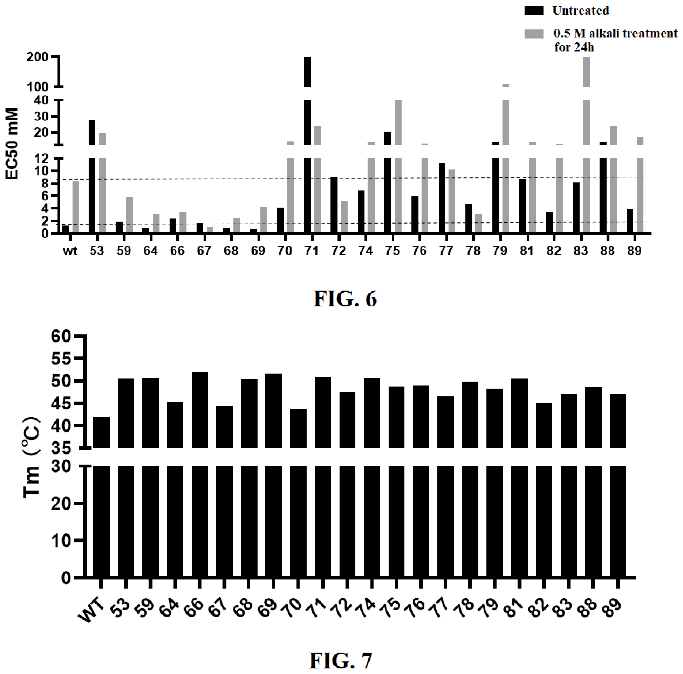

Taking the protein fragmentation status after alkali treatment as the primary screening index, 40 multi-point mutants were subjected to alkali treatment (0.5M NaOH, 24h), and the summarized SDS-PAGE detection results are shown in Fig. 5. ELISA comparisons of affinity and alkali resistance for 24h were performed on 21 mutants (M78, M74, M69, M53, M67, M88, M68, M82, M89, M66, M76, M64, M83, M81, M77, M71, M79, M70, M72, M75, M59) with the proportion of degraded bands lower than 50%. The results are shown in Fig. 6.

By comparing the absolute values of the ELISA detection values of the above-mentioned multi-point mutants after alkali treatment, it can be determined that 21 multi-point mutants still have binding ability, among which 8 multi-point mutants (M67, M68, M78, M64, M66, M69, M72 and M59) still maintain strong affinity after alkali treatment.

### Example 5: Investigation of heat resistance of second-round designed proteins (multi-point mutants)

The heat resistance of the 21 multi-point mutants was investigated. The heat resistance test method is as described in Example 3. The results are shown in Fig. 7. The Tm values of the above 21 multi-point mutants were all increased to varying degrees compared with WT, that is, the heat resistance of the above mutants was also improved to varying degrees, which indirectly indicates the further improvement of the stability of multi-point mutants.

### Example 6: Preparation of preferred molecular samples and their conjugated affinity media

After screening the selected proteins from the first and second rounds of sequence design for molecular alkali resistance and heat resistance, we selected 6 preferred molecules for further verification: M20 and M45 from the first round and M67, M68, M74 and M78 from the second round.

After protein sequence design and site-directed mutagenesis, the sequences were synthesized and constructed, then transformed into host cells to express target proteins. Following fermentation, nickel affinity chromatography, and ultrafiltration centrifugation, the proteins were subjected to disulfide bond renaturation using urea, oxidizing and reducing agents, followed by fine purification. The resulting purified proteins were adopted as the modified target proteins for subsequent analysis. Protein purity was tested, and samples with a purity >90% were used for verification experiments.

The protein was subjected to differential scanning calorimetry (DSC) to determine its Tm. The protein was incubated with 0.1M NaOH and 0.5M NaOH, followed by pH neutralization. The supernatant was collected by centrifugation, and the protein purity was determined using SDS-PAGE. The protein was provided to a commissioned company specializing in affinity media development for the preparation of preferred molecule-conjugated affinity media. The affinity media were incubated with 0.1M NaOH and 0.5M NaOH for different durations, and the dynamic binding capacity at 10% breakthrough of the affinity media before and after alkali treatment was evaluated. Meanwhile, the unmodified protein was processed and its corresponding media were prepared for comparison with those of the preferred molecules. An increase in Tm of the optimized molecules indicates improved protein thermostability; reduced degraded bands or smearing degree in electrophoresis results after alkali treatment demonstrates enhanced alkali resistance of the molecules. The conjugated media of alkali-treated preferred molecules exhibit a lower reduction rate of 10% dynamic binding capacity, which proves that the preferred molecules have better alkali resistance and can effectively improve the alkali resistance of their affinity media.

### Example 7: Investigation on alkali resistance of preferred molecules

The proteins before and after modification were incubated with 0.1M NaOH and 0.5M NaOH, respectively. The incubation time in the 0.1M NaOH system was set at 0h and 24h; the incubation time in the 0.5M NaOH system was set at 0h, 6h, 16h and 24h. After incubation, acetic acid was used to neutralize the solution to a neutral pH. The samples were centrifuged at 4000 RPM and 4°C for 20 min, and the supernatants were collected. After concentration determination, electrophoretic purity analysis was performed. The protein after alkali treatment was detected by SDS-PAGE, which provides the most direct way to observe the protein's alkali resistance.

The sample was diluted with ultrapure water, and 2× non-reducing sample buffer was added at a ratio of 1:1. The sample was then heated in a metal bath at 60°C for 5 min. Electrophoresis was carried out using a 12% precast gel under a constant voltage of 100V until bromophenol blue migrated to the lowest point of the gel (at the edge of the gel), then the electrophoresis was stopped. Staining and destaining were conducted. A constant protein loading amount of 5µg was applied to test the control and proteins treated with alkali for different durations. The gel was scanned and analyzed with a corresponding scanner, and calculated using the area normalization method.

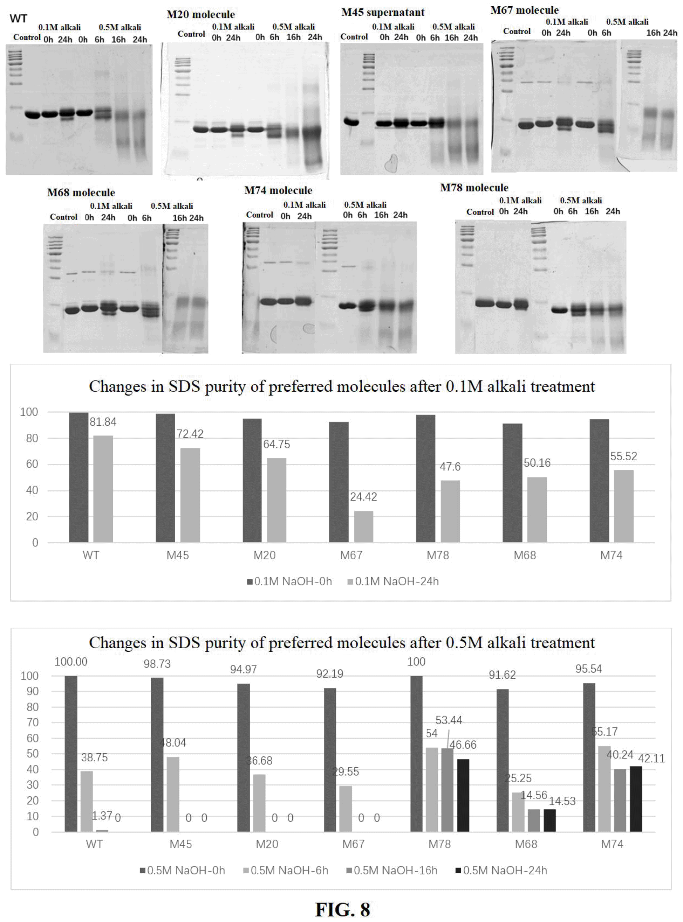

The protein electrophoresis results and their corresponding purity changes are shown in Fig. 8. According to these results, after treatment with 0.1M NaOH for 24h, the target bands of protein molecules such as M45, M74 and M78 exhibited fewer degraded bands, demonstrating improved alkali resistance compared with the unmodified protein. When the protein molecules were treated with 0.5M NaOH, some preferred mutant proteins (such as M20, M74 and M78) showed a higher proportion of target protein bands and reduced smearing after alkali treatment, exhibiting enhanced alkali stability compared with the unmodified protein. The data analyzed by the SDS-PAGE purity analyzer have limitations and are for reference only. Electrophoretograms can directly visualize molecules with excellent alkali resistance.

### Example 8: Investigation on heat resistance of preferred molecules

### Heat resistance was tested using a differential scanning calorimeter (DSC).

Sample preparation: An appropriate amount of each test sample was taken and diluted to the same concentration with buffer solution, then mixed evenly for later use. Parameter settings: initial temperature, final temperature, heating rate, equilibration time, stirring cycles, sample loading volume.

The results are shown in Table 2. The Tm values of the above 6 preferred molecules were equivalent to or increased to varying degrees compared with the WT.

**Table 2 DSC verification results of preferred molecules**

| Preferred verified molecules | Tm (°C) | Temperature increase (°C) |
|---|---|---|
| WT | 75.78 | - |
| M45 | 75.83 | 0.05 |
| M20 | 80.46 | 4.68 |
| M67 | 76.26 | 0.48 |
| M78 | 81.69 | 5.91 |
| M74 | 87.64 | 11.86 |
| M68 | 79.09 | 3.31 |

### Example 9: Investigation on alkali resistance of preferred molecule-conjugated media

The prepared preferred molecules were provided to the medium manufacturer, who prepared affinity media conjugated with the preferred molecules using the same conjugation method and consistent protein coupling ratio. The prepared medium was incubated with 0.1M NaOH for 24h and 32h, and with 0.5M alkali for 6h and 24h. Dynamic binding capacity (DBC) at 10% breakthrough was determined using GH stock solution before and after alkali incubation.

Experimental sample: GH stock solution, diluted to 5 mg/ml. Chromatography column model: Bestchrom 10/20, chromatography medium volume: 7.85 ml. The retention time for the chromatography experiment was 5 min. The binding capacity determination procedure is as follows:

**Table 3 Determination procedure for binding capacity in alkali resistance investigation of conjugated media**

| Step | Content | Flow rate (ml/min) | Volume (CV) |
|---|---|---|---|
| CIP | 0.1M NaOH | 3 | 3 |
| Equilibration | Add 20mM Tris-150mM NaCl (pH 7.5) to achieve the same pH and conductivity as the equilibration buffer | 3 | 5-7 |
| Loading | Dilute the sample with GH stock solution, stop loading when the UV reaches 10% breakthrough UV value of the loaded sample | 1.57 | - |
| Equilibration | Add 20mM Tris-150mM NaCl (pH 7.5) to achieve the same pH and conductivity as the equilibration buffer | 1.57 | 5 |
| Elution | 100mM HAC-NaAC (pH 3.7) | 3 | 6 |
| Regeneration | 1M HAC | 3 | 3 |
| Equilibration | 20mM Tris-150mM NaCl (pH 7.5) | 3 | 3 |
| Cleaning | 0.1M NaOH | 3 | 3 |
| Equilibration | Add 20mM Tris-150mM NaCl (pH7.5) to achieve the same pH and conductivity as the equilibration buffer | 3 | 5 |

**Table 4 Dynamic binding capacity under 10% breakthrough conditions after incubation in 0.1M NaOH for 24h and 32h**

| 0.1M NaOH | WT | M20 | M45 | M67 | M78 | M68 | M74 |
|---|---|---|---|---|---|---|---|
| 0h | 22.3 | 19.58 | 18.19 | 15.91 | 16.3 | 15.9 | 15.03 |
| 24h | 16.16 | 17.97 | 17.38 | 16.17 | 16.30 | 16.17 | 15.77 |
| 32h | 14.71 | 16.92 | 16.23 | 14.89 | 15.42 | 15.44 | 15.38 |

**Table 5 Dynamic binding capacity under 10% breakthrough conditions after incubation in 0.5M NaOH for 6h and 24h**

| 0.5MNaOH | WT | M20 | M45 | M67 | M78 | M68 | M74 |
|---|---|---|---|---|---|---|---|
| 0h | 22.3 | 19.58 | 18.19 | 15.91 | 16.3 | 15.34 | 14.66 |
| 6h | 5.78 | 14.29 | 7.60 | 10.15 | 13.49 | 12.20 | 13.20 |
| 24h | 3.39 | 10.15 | 4.29 | 4.23 | 9.06 | 5.41 | 8.24 |

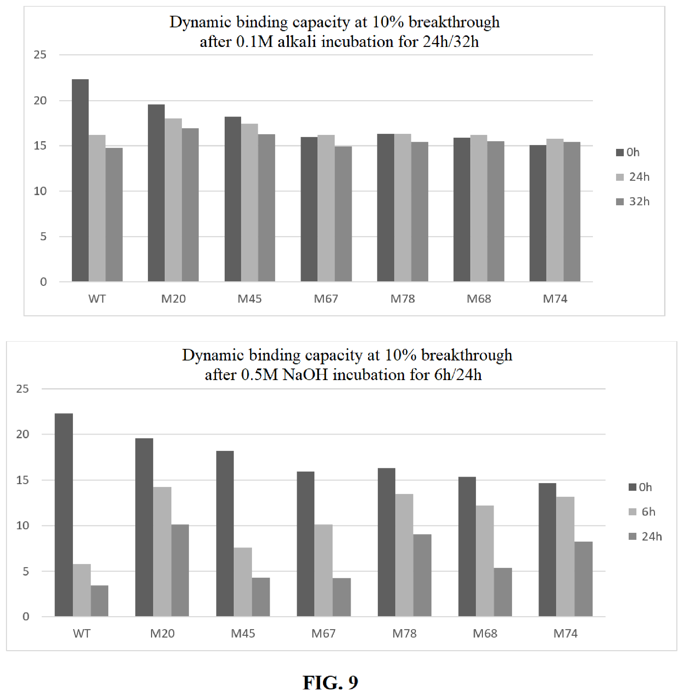

The results are presented in Fig. 9 and Tables 4-5. For the above 6 preferred molecule-conjugated media, their binding capacity was improved to varying degrees after alkaline incubation compared with the WT. When treated with 0.1M NaOH for 32h, the DBC decay rate decreased from 35% to 0-14%; the Top 3 molecules M20, M74, and M78 exhibited a value decreasing to no more than 6%; when treated with 0.5M NaOH for 24h, residual DBC dropped from 85% to 44-77%, while the Top 3 molecules maintained a decrease of no more than 49%. This experiment mainly considers the decreasing trend of DBC. Since the media coupling process is not optimized, the absolute value of DBC at 0 is not of reference significance.

Alkali resistance and stability verification were performed on the 6 preferred molecules and their conjugated media. The engineered proteins show enhanced alkali resistance and heat resistance in comparison with the WT molecule.

### Summary:

Sequence optimization was performed based on the second-round molecules and the first-round preferred molecules. A total of 35 preferred molecules were obtained. The sequence information of the 35 preferred mutants and the control (WT) is summarized in Table 6.

**Table 6 Sequence information of WT molecules and preferred molecules**

| Designation | Mutation site (Based on kabat numbering system) | C-terminal polypeptide information | | | SEQ ID NO: |
|---|---|---|---|---|---|
| WT | - | DDDDK | SGGGGS | HHHHHH | 1 |
| M8 (First round) | D102Y | DDDDK | SGGGGS | HHHHHH | 2 |
| M11 (First round) | P105Q | DDDDK | SGGGGS | HHHHHH | 3 |
| M20 (First round) | L48V | DDDDK | SGGGGS | HHHHHH | 4 |
| M22 (First round) | L11K | DDDDK | SGGGGS | HHHHHH | 5 |
| M24 (First round) | W100gF | DDDDK | SGGGGS | HHHHHH | 6 |
| M25 (First round) | V101D | DDDDK | SGGGGS | HHHHHH | 7 |
| M27 (First round) | P28T | DDDDK | SGGGGS | HHHHHH | 8 |
| M28 (First round) | R19T | DDDDK | SGGGGS | HHHHHH | 9 |
| M29 (First round) | T56K | DDDDK | SGGGGS | HHHHHH | 10 |
| M31 (First round) | R100eE | DDDDK | SGGGGS | HHHHHH | 11 |
| M33 (First round) | A55D | DDDDK | SGGGGS | HHHHHH | 12 |
| M34 (First round) | N76S | DDDDK | SGGGGS | HHHHHH | 13 |
| M45 (First round) | - | DDDDK | SGGRGS | HHHHHH | 14 |
| M50 (First round) | A14P | DDDDK | SGGGGS | HHHHHH | 15 |
| M53 (Second round) | A55D; R100eE | DDDDK | SGGGGS | HHHHHH | 16 |
| M59 (Second round) | P28T; A55D | DDDDK | SGGGGS | HHHHHH | 17 |
| M64 (Second round) | A14P | DDDDK | SGGRGS | HHHHHH | 18 |
| M66 (Second round) | A14P; P28T | DDDDK | SGGGGS | HHHHHH | 19 |
| M67 (Second round) | A14P; P105Q | DDDDK | SGGGGS | HHHHHH | 20 |
| M68 (Second round) | A14P; R19T | DDDDK | SGGGGS | HHHHHH | 21 |
| M69 (Second round) | P28T | DDDDK | SGGRGS | HHHHHH | 22 |
| M70 (Second round) | A14P; R100eE | DDDDK | SGGGGS | HHHHHH | 23 |
| M71 (Second round) | A55D; R100eE; P105Q | DDDDK | SGGGGS | HHHHHH | 24 |
| M72 (Second round) | A55D; W100gF; D102Y | DDDDK | SGGGGS | HHHHHH | 25 |
| M74 (Second round) | A55D; R100eE | DDDDK | SGGRGS | HHHHHH | 26 |
| M75 (Second round) | A55D; R100eE; D102Y | DDDDK | SGGGGS | HHHHHH | 27 |
| M76 (Second round) | A55D; W100gF | DDDDK | SGGRGS | HHHHHH | 28 |
| M77 (Second round) | A55D; R100eE; W100gF | DDDDK | SGGGGS | HHHHHH | 29 |
| M78 (Second round) | A55D; D102Y | DDDDK | SGGRGS | HHHHHH | 30 |
| M79 (Second round) | L48V; A55D; R100eE | DDDDK | SGGGGS | HHHHHH | 31 |
| M81 (Second round) | A55D; R100eE; P105Q | DDDDK | SGGRGS | HHHHHH | 32 |
| M82 (Second round) | A55D; R100eE; D102Y | DDDDK | SGGRGS | HHHHHH | 33 |
| M83 (Second round) | A55D; R100eE; W100gF; P105Q | DDDDK | SGGGGS | HHHHHH | 34 |
| M88 (Second round) | L48V; A55D; R100eE | DDDDK | SGGRGS | HHHHHH | 35 |
| M89 (Second round) | A55D; R100eE; W100gF | DDDDK | SGGRGS | HHHHHH | 36 |

Although the specific embodiments of the present invention have been described in detail, those skilled in the art will understand that various modifications and changes may be made to the details based on all the teachings published, and these changes are all within the scope of protection of the present invention. The entire scope of the present invention is given by the appended claims and any equivalents thereof.

## Claims

1. A single-domain antibody capable of specifically binding to a growth hormone (GH), or an antigen-binding fragment thereof, wherein the single-domain antibody or antigen-binding fragment thereof comprises complementarity-determining regions (CDRs) and framework regions (FRs), and has one or more features selected from the group consisting of:
(i) the amino acid residue at position 102 is Y;
(ii) the amino acid residue at position 105 is Q;
(iii) the amino acid residue at position 48 is V;
(iv) the amino acid residue at position 11 is K;
(v) the amino acid residue at position 100g is F;
(vi) the amino acid residue at position 101 is D;
(vii) the amino acid residue at position 28 is T;
(viii) the amino acid residue at position 19 is T;
(ix) the amino acid residue at position 56 is K;
(x) the amino acid residue at position 100e is E;
(xi) the amino acid residue at position 55 is D;
(xii) the amino acid residue at position 76 is S;
(xiii) the amino acid residue at position 14 is P;
wherein the positions are defined by the kabat numbering system.

2. The single-domain antibody or antigen-binding fragment thereof of claim 1, having one or more features selected from the group consisting of:
(a) the amino acid residue at position 102 is Y;
(b) the amino acid residue at position 105 is Q;
(c) the amino acid residue at position 48 is V;
(d) the amino acid residue at position 100g is F;
(e) the amino acid residue at position 28 is T;
(f) the amino acid residue at position 19 is T;
(g) the amino acid residue at position 100e is E;
(h) the amino acid residue at position 55 is D;
(i) the amino acid residue at position 14 is P;
wherein the positions are defined by the kabat numbering system;
preferably, the single-domain antibody or antigen-binding fragment thereof has features selected from the group consisting of:
(1) the amino acid residue at position 55 is D, and the amino acid residue at position 100e is E;
(2) the amino acid residue at position 28 is T, and the amino acid residue at position 55 is D;
(3) the amino acid residue at position 14 is P, and the amino acid residue at position 28 is T;
(4) the amino acid residue at position 14 is P, and the amino acid residue at position 105 is Q;
(5) the amino acid residue at position 14 is P, and the amino acid residue at position 19 is T;
(6) the amino acid residue at position 14 is P, and the amino acid residue at position 100e is E;
(7) the amino acid residue at position 55 is D, the amino acid residue at position 100e is E, and the amino acid residue at position 105 is Q;
(8) the amino acid residue at position 55 is D, the amino acid residue at position 100g is F, and the amino acid residue at position 102 is Y;
(9) the amino acid residue at position 55 is D, the amino acid residue at position 100e is E, and the amino acid residue at position 102 is Y;
(10) the amino acid residue at position 55 is D, and the amino acid residue at position 100g is F;
(11) the amino acid residue at position 55 is D, the amino acid residue at position 100e is E, and the amino acid residue at position 100g is F;
(12) the amino acid residue at position 55 is D, and the amino acid residue at position 102 is Y;
(13) the amino acid residue at position 48 is V, the amino acid residue at position 55 is D, and the amino acid residue at position 100e is E;
(14) the amino acid residue at position 55 is D, the amino acid residue at position 100e is E, and the amino acid residue at position 105 is Q;
(15) the amino acid residue at position 55 is D, the amino acid residue at position 100e is E, the amino acid residue at position 100g is F, and the amino acid residue at position 105 is Q;
wherein the positions are defined by the kabat numbering system.

3. The single-domain antibody or antigen-binding fragment thereof of claim 1 or 2, wherein the single-domain antibody or antigen-binding fragment thereof has any one of the following CDR1, CDR2 and CDR3:
(1) CDR1 as set forth in SEQ ID NO: 42; CDR2 as set forth in SEQ ID NO: 43; and CDR3 as set forth in SEQ ID NO: 47;
(2) CDR1 as set forth in SEQ ID NO: 42; CDR2 as set forth in SEQ ID NO: 43; and CDR3 as set forth in SEQ ID NO: 48;
(3) CDR1 as set forth in SEQ ID NO: 42; CDR2 as set forth in SEQ ID NO: 43; and CDR3 as set forth in SEQ ID NO: 49;
(4) CDR1 as set forth in SEQ ID NO: 42; CDR2 as set forth in SEQ ID NO: 44; and CDR3 as set forth in SEQ ID NO: 46;
(5) CDR1 as set forth in SEQ ID NO: 42; CDR2 as set forth in SEQ ID NO: 43; and CDR3 as set forth in SEQ ID NO: 50;
(6) CDR1 as set forth in SEQ ID NO: 42; CDR2 as set forth in SEQ ID NO: 45; and CDR3 as set forth in SEQ ID NO: 46;
(7) CDR1 as set forth in SEQ ID NO: 42; CDR2 as set forth in SEQ ID NO: 45; and CDR3 as set forth in SEQ ID NO: 50;
(8) CDR1 as set forth in SEQ ID NO: 42; CDR2 as set forth in SEQ ID NO: 45; and CDR3 as set forth in SEQ ID NO: 51;
(9) CDR1 as set forth in SEQ ID NO: 42; CDR2 as set forth in SEQ ID NO: 45; and CDR3 as set forth in SEQ ID NO: 52;
(10) CDR1 as set forth in SEQ ID NO: 42; CDR2 as set forth in SEQ ID NO: 45; and CDR3 as set forth in SEQ ID NO: 48;
(11) CDR1 as set forth in SEQ ID NO: 42; CDR2 as set forth in SEQ ID NO: 45; and CDR3 as set forth in SEQ ID NO: 53;
or,
(12) CDR1 as set forth in SEQ ID NO: 42; CDR2 as set forth in SEQ ID NO: 45; and CDR3 as set forth in SEQ ID NO: 47.

4. The single-domain antibody or antigen-binding fragment thereof of claim 1, wherein compared with the sequence as set forth in SEQ ID NO: 37, the single-domain antibody has one or more substitution mutations selected from the group consisting of:
D102Y, P105Q, L48V, L11K, W100gF, V101D, P28T, R19T, T56K, R100eE, A55D, N76S, A14P;
wherein the positions of the mutations are defined by the kabat numbering system;
preferably, compared with the sequence as set forth in SEQ ID NO: 37, the single-domain antibody has one or more substitution mutations selected from the group consisting of:
D102Y, P105Q, L48V, W100gF, P28T, R19T, R100eE, A55D, A14P;
wherein the positions of the mutations are defined by the kabat numbering system;
preferably, compared with the sequence as set forth in SEQ ID NO: 37, the single-domain antibody has:
(1) substitution of A at position 55 with D, and substitution of R at position 100e with E (A55D and R100eE);
(2) substitution of P at position 28 with T, and substitution of A at position 55 with D (P28T and A55D);
(3) substitution of A at position 14 with P, and substitution of P at position 28 with T (A14P and P28T);
(4) substitution of A at position 14 with P, and substitution of P at position 105 with Q (A14P and P105Q);
(5) substitution of A at position 14 with P, and substitution of R at position 19 with T (A14P and R19T);
(6) substitution of A at position 14 with P, and substitution of R at position 100e with E (A14P and R100eE);
(7) substitution of A at position 55 with D, substitution of R at position 100e with E, and substitution of P at position 105 with Q (A55D, R100eE and P105Q);
(8) substitution of A at position 55 with D, substitution of W at position 100g with F, and substitution of D at position 102 with Y (A55D, W100gF and D102Y);
(9) substitution of A at position 55 with D, substitution of R at position 100e with E, and substitution of D at position 102 with Y (A55D, R100eE and D102Y);
(10) substitution of A at position 55 with D, and substitution of W at position 100g with F (A55D and W100gF);
(11) substitution of A at position 55 with D, substitution of R at position 100e with E, and substitution of W at position 100g with F (A55D, R100eE and W100gF);
(12) substitution of A at position 55 with D, and substitution of D at position 102 with Y (A55D and D102Y);
(13) substitution of L at position 48 with V, substitution of A at position 55 with D, and substitution of R at position 100e with E (L48V, A55D and R100eE);
(14) substitution of A at position 55 with D, substitution of R at position 100e with E, and substitution of P at position 105 with Q (A55D, R100eE and P105Q);
or,
(15) substitution of A at position 55 with D, substitution of R at position 100e with E, substitution of W at position 100g with F, and substitution of P at position 105 with Q (A55D, R100eE, W100gF and P105Q);
wherein the positions are defined by the kabat numbering system.

5. The single-domain antibody or antigen-binding fragment thereof of any one of claims 1 to 4, comprising an amino acid sequence selected from the group consisting of:
(1) the amino acid sequence as set forth in positions 2-125 or positions 1-125 of SEQ ID NO: 2;
(2) the amino acid sequence as set forth in positions 2-125 or positions 1-125 of SEQ ID NO: 3;
(3) the amino acid sequence as set forth in positions 2-125 or positions 1-125 of SEQ ID NO: 4;
(4) the amino acid sequence as set forth in positions 2-125 or positions 1-125 of SEQ ID NO: 5;
(5) the amino acid sequence as set forth in positions 2-125 or positions 1-125 of SEQ ID NO: 6;
(6) the amino acid sequence as set forth in positions 2-125 or positions 1-125 of SEQ ID NO: 7;
(7) the amino acid sequence as set forth in positions 2-125 or positions 1-125 of SEQ ID NO: 8;
(8) the amino acid sequence as set forth in positions 2-125 or positions 1-125 of SEQ ID NO: 9;
(9) the amino acid sequence as set forth in positions 2-125 or positions 1-125 of SEQ ID NO: 10;
(10) the amino acid sequence as set forth in positions 2-125 or positions 1-125 of SEQ ID NO: 11;
(11) the amino acid sequence as set forth in positions 2-125 or positions 1-125 of SEQ ID NO: 12;
(12) the amino acid sequence as set forth in positions 2-125 or positions 1-125 of SEQ ID NO: 13;
(13) the amino acid sequence as set forth in positions 2-125 or positions 1-125 of SEQ ID NO: 15;
(14) the amino acid sequence as set forth in positions 2-125 or positions 1-125 of SEQ ID NO: 16;
(15) the amino acid sequence as set forth in positions 2-125 or positions 1-125 of SEQ ID NO: 17;
(16) the amino acid sequence as set forth in positions 2-125 or positions 1-125 of SEQ ID NO: 18;
(17) the amino acid sequence as set forth in positions 2-125 or positions 1-125 of SEQ ID NO: 19;
(18) the amino acid sequence as set forth in positions 2-125 or positions 1-125 of SEQ ID NO: 20;
(19) the amino acid sequence as set forth in positions 2-125 or positions 1-125 of SEQ ID NO: 21;
(20) the amino acid sequence as set forth in positions 2-125 or positions 1-125 of SEQ ID NO: 22;
(21) the amino acid sequence as set forth in positions 2-125 or positions 1-125 of SEQ ID NO: 23;
(22) the amino acid sequence as set forth in positions 2-125 or positions 1-125 of SEQ ID NO: 24;
(23) the amino acid sequence as set forth in positions 2-125 or positions 1-125 of SEQ ID NO: 25;
(24) the amino acid sequence as set forth in positions 2-125 or positions 1-125 of SEQ ID NO: 26;
(25) the amino acid sequence as set forth in positions 2-125 or positions 1-125 of SEQ ID NO: 27;
(26) the amino acid sequence as set forth in positions 2-125 or positions 1-125 of SEQ ID NO: 28;
(27) the amino acid sequence as set forth in positions 2-125 or positions 1-125 of SEQ ID NO: 29;
(28) the amino acid sequence as set forth in positions 2-125 or positions 1-125 of SEQ ID NO: 30;
(29) the amino acid sequence as set forth in positions 2-125 or positions 1-125 of SEQ ID NO: 31;
(30) the amino acid sequence as set forth in positions 2-125 or positions 1-125 of SEQ ID NO: 32;
(31) the amino acid sequence as set forth in positions 2-125 or positions 1-125 of SEQ ID NO: 33;
(32) the amino acid sequence as set forth in positions 2-125 or positions 1-125 of SEQ ID NO: 34;
(33) the amino acid sequence as set forth in positions 2-125 or positions 1-125 of SEQ ID NO: 35;
(34) the amino acid sequence as set forth in positions 2-125 or positions 1-125 of SEQ ID NO: 36.

6. A single-domain antibody capable of specifically binding to a growth hormone (GH) or an antigen-binding fragment thereof, wherein the single-domain antibody or antigen-binding fragment thereof comprises:
(1) CDR1 as set forth in SEQ ID NO: 42; CDR2 as set forth in SEQ ID NO: 43; and CDR3 as set forth in SEQ ID NO: 47;
(2) CDR1 as set forth in SEQ ID NO: 42; CDR2 as set forth in SEQ ID NO: 43; and CDR3 as set forth in SEQ ID NO: 48;
(3) CDR1 as set forth in SEQ ID NO: 42; CDR2 as set forth in SEQ ID NO: 43; and CDR3 as set forth in SEQ ID NO: 49;
(4) CDR1 as set forth in SEQ ID NO: 42; CDR2 as set forth in SEQ ID NO: 44; and CDR3 as set forth in SEQ ID NO: 46;
(5) CDR1 as set forth in SEQ ID NO: 42; CDR2 as set forth in SEQ ID NO: 43; and CDR3 as set forth in SEQ ID NO: 50;
(6) CDR1 as set forth in SEQ ID NO: 42; CDR2 as set forth in SEQ ID NO: 45; and CDR3 as set forth in SEQ ID NO: 46;
(7) CDR1 as set forth in SEQ ID NO: 42; CDR2 as set forth in SEQ ID NO: 45; and CDR3 as set forth in SEQ ID NO: 50;
(8) CDR1 as set forth in SEQ ID NO: 42; CDR2 as set forth in SEQ ID NO: 45; and CDR3 as set forth in SEQ ID NO: 51;
(9) CDR1 as set forth in SEQ ID NO: 42; CDR2 as set forth in SEQ ID NO: 45; and CDR3 as set forth in SEQ ID NO: 52;
(10) CDR1 as set forth in SEQ ID NO: 42; CDR2 as set forth in SEQ ID NO: 45; and CDR3 as set forth in SEQ ID NO: 48;
(11) CDR1 as set forth in SEQ ID NO: 42; CDR2 as set forth in SEQ ID NO: 45; and CDR3 as set forth in SEQ ID NO: 53;
or,
(12) CDR1 as set forth in SEQ ID NO: 42; CDR2 as set forth in SEQ ID NO: 45; and CDR3 as set forth in SEQ ID NO: 47;
preferably, the CDRs are defined by the kabat numbering system;
preferably, the single-domain antibody or antigen-binding fragment thereof comprises an amino acid sequence selected from the group consisting of:
(i) the amino acid sequence as set forth in positions 2-125 or positions 1-125 of SEQ ID NO: 2;
(ii) the amino acid sequence as set forth in positions 2-125 or positions 1-125 of SEQ ID NO: 6;
(iii) the amino acid sequence as set forth in positions 2-125 or positions 1-125 of SEQ ID NO: 7;
(iv) the amino acid sequence as set forth in positions 2-125 or positions 1-125 of SEQ ID NO: 10;
(v) the amino acid sequence as set forth in positions 2-125 or positions 1-125 of SEQ ID NO: 11;
(vi) the amino acid sequence as set forth in positions 2-125 or positions 1-125 of SEQ ID NO: 12;
(vii) the amino acid sequence as set forth in positions 2-125 or positions 1-125 of SEQ ID NO: 16;
(viii) the amino acid sequence as set forth in positions 2-125 or positions 1-125 of SEQ ID NO: 17;
(ix) the amino acid sequence as set forth in positions 2-125 or positions 1-125 of SEQ ID NO: 23;
(x) the amino acid sequence as set forth in positions 2-125 or positions 1-125 of SEQ ID NO: 24;
(xi) the amino acid sequence as set forth in positions 2-125 or positions 1-125 of SEQ ID NO: 25;
(xii) the amino acid sequence as set forth in positions 2-125 or positions 1-125 of SEQ ID NO: 26;
(xiii) the amino acid sequence as set forth in positions 2-125 or positions 1-125 of SEQ ID NO: 27;
(xiv) the amino acid sequence as set forth in positions 2-125 or positions 1-125 of SEQ ID NO: 28;
(xv) the amino acid sequence as set forth in positions 2-125 or positions 1-125 of SEQ ID NO: 29;
(xvi) the amino acid sequence as set forth in positions 2-125 or positions 1-125 of SEQ ID NO: 30;
(xvii) the amino acid sequence as set forth in positions 2-125 or positions 1-125 of SEQ ID NO: 31;
(xviii) the amino acid sequence as set forth in positions 2-125 or positions 1-125 of SEQ ID NO: 32;
(xix) the amino acid sequence as set forth in positions 2-125 or positions 1-125 of SEQ ID NO: 33;
(xx) the amino acid sequence as set forth in positions 2-125 or positions 1-125 of SEQ ID NO: 34;
(xxi) the amino acid sequence as set forth in positions 2-125 or positions 1-125 of SEQ ID NO: 35;
(xxii) the amino acid sequence as set forth in positions 2-125 or positions 1-125 of SEQ ID NO: 36.

7. A polypeptide construct capable of specifically binding to a growth hormone (GH), comprising the single-domain antibody or antigen-binding fragment thereof of any one of claims 1 to 6, and an additional polypeptide;
preferably, the polypeptide construct is a fusion protein;
preferably, the additional polypeptide is optionally linked to the N-terminus or C-terminus of the single-domain antibody or antigen-binding fragment thereof via a linker;
preferably, the additional polypeptide is selected from the group consisting of protein tags (e.g., His tags, such as HHHHHH or HHHHHHHH), protease recognition sequences (e.g., EK protease recognition sequences, such as DDDDK), peptide linkers (e.g., flexible peptide linkers; e.g., peptide linkers comprising one or more glycine (G) and/or serine (S)), or any combination thereof;
preferably, the polypeptide construct comprises, in order from the N-terminus to the C-terminus: the single-domain antibody or antigen-binding fragment thereof of any one of claims 1 to 6, an EK protease recognition sequence (e.g., DDDDK), a flexible peptide linker (e.g., the peptide linker as set forth in SEQ ID NO: 40), and an optional His tag (e.g., HHHHHH or HHHHHHHH);
preferably, the polypeptide construct comprises an amino acid sequence selected from the group consisting of:
(1) the amino acid sequence as set forth in SEQ ID NO: 2, or the amino acid sequence as set forth in positions 1-136, or positions 2-136, or positions 2-142 of SEQ ID NO: 2;
(2) the amino acid sequence as set forth in SEQ ID NO: 3, or the amino acid sequence as set forth in positions 1-136, or positions 2-136, or positions 2-142 of SEQ ID NO: 3;
(3) the amino acid sequence as set forth in SEQ ID NO: 4, or the amino acid sequence as set forth in positions 1-136, or positions 2-136, or positions 2-142 of SEQ ID NO: 4;
(4) the amino acid sequence as set forth in SEQ ID NO: 5, or the amino acid sequence as set forth in positions 1-136, or positions 2-136, or positions 2-142 of SEQ ID NO: 5;
(5) the amino acid sequence as set forth in SEQ ID NO: 6, or the amino acid sequence as set forth in positions 1-136, or positions 2-136, or positions 2-142 of SEQ ID NO: 6;
(6) the amino acid sequence as set forth in SEQ ID NO: 7, or the amino acid sequence as set forth in positions 1-136, or positions 2-136, or positions 2-142 of SEQ ID NO: 7;
(7) the amino acid sequence as set forth in SEQ ID NO: 8, or the amino acid sequence as set forth in positions 1-136, or positions 2-136, or positions 2-142 of SEQ ID NO: 8;
(8) the amino acid sequence as set forth in SEQ ID NO: 9, or the amino acid sequence as set forth in positions 1-136, or positions 2-136, or positions 2-142 of SEQ ID NO: 9;
(9) the amino acid sequence as set forth in SEQ ID NO: 10, or the amino acid sequence as set forth in positions 1-136, or positions 2-136, or positions 2-142 of SEQ ID NO: 10;
(10) the amino acid sequence as set forth in SEQ ID NO: 11, or the amino acid sequence as set forth in positions 1-136, or positions 2-136, or positions 2-142 of SEQ ID NO: 11;
(11) the amino acid sequence as set forth in SEQ ID NO: 12, or the amino acid sequence as set forth in positions 1-136, or positions 2-136, or positions 2-142 of SEQ ID NO: 12;
(12) the amino acid sequence as set forth in SEQ ID NO: 13, or the amino acid sequence as set forth in positions 1-136, or positions 2-136, or positions 2-142 of SEQ ID NO: 13;
(13) the amino acid sequence as set forth in SEQ ID NO: 15, or the amino acid sequence as set forth in positions 1-136, or positions 2-136, or positions 2-142 of SEQ ID NO: 15;
(14) the amino acid sequence as set forth in SEQ ID NO: 16, or the amino acid sequence as set forth in positions 1-136, or positions 2-136, or positions 2-142 of SEQ ID NO: 16;
(15) the amino acid sequence as set forth in SEQ ID NO: 17, or the amino acid sequence as set forth in positions 1-136, or positions 2-136, or positions 2-142 of SEQ ID NO: 17;
(16) the amino acid sequence as set forth in SEQ ID NO: 19, or the amino acid sequence as set forth in positions 1-136, or positions 2-136, or positions 2-142 of SEQ ID NO: 19;
(17) the amino acid sequence as set forth in SEQ ID NO: 20, or the amino acid sequence as set forth in positions 1-136, or positions 2-136, or positions 2-142 of SEQ ID NO: 20;
(18) the amino acid sequence as set forth in SEQ ID NO: 21, or the amino acid sequence as set forth in positions 1-136, or positions 2-136, or positions 2-142 of SEQ ID NO: 21;
(19) the amino acid sequence as set forth in SEQ ID NO: 23, or the amino acid sequence as set forth in positions 1-136, or positions 2-136, or positions 2-142 of SEQ ID NO: 23;
(20) the amino acid sequence as set forth in SEQ ID NO: 24, or the amino acid sequence as set forth in positions 1-136, or positions 2-136, or positions 2-142 of SEQ ID NO: 24;
(21) the amino acid sequence as set forth in SEQ ID NO: 25, or the amino acid sequence as set forth in positions 1-136, or positions 2-136, or positions 2-142 of SEQ ID NO: 25;
(22) the amino acid sequence as set forth in SEQ ID NO: 27, or the amino acid sequence as set forth in positions 1-136, or positions 2-136, or positions 2-142 of SEQ ID NO: 27;
(23) the amino acid sequence as set forth in SEQ ID NO: 29, or the amino acid sequence as set forth in positions 1-136, or positions 2-136, or positions 2-142 of SEQ ID NO: 29;
(24) the amino acid sequence as set forth in SEQ ID NO: 31, or the amino acid sequence as set forth in positions 1-136, or positions 2-136, or positions 2-142 of SEQ ID NO: 31;
(25) the amino acid sequence as set forth in SEQ ID NO: 34, or the amino acid sequence as set forth in positions 1-136, or positions 2-136, or positions 2-142 of SEQ ID NO: 34.

8. A polypeptide construct capable of specifically binding to a growth hormone (GH), comprising a single-domain antibody capable of specifically binding to GH or an antigen-binding fragment thereof, and a polypeptide as set forth in SEQ ID NO: 41.

9. The polypeptide construct of claim 8, wherein the single-domain antibody capable of specifically binding to GH or antigen-binding fragment thereof is selected from: (i) the single-domain antibody or antigen-binding fragment thereof of any one of claims 1 to 6, and (ii) the single-domain antibody as set forth in SEQ ID NO: 37 or antigen-binding fragment thereof.

10. The polypeptide construct of claim 8 or 9, wherein the polypeptide construct is a fusion protein;
preferably, the polypeptide construct comprises the polypeptide as set forth in SEQ ID NO: 41 at the C-terminus of the single-domain antibody capable of specifically binding to GH or antigen-binding fragment thereof;
preferably, the polypeptide construct further comprises an EK protease recognition sequence (e.g., DDDDK) and/or a His tag (e.g., HHHHHH or HHHHHHHH);
preferably, the polypeptide construct comprises, in order from the N-terminus to the C-terminus: the single-domain antibody capable of specifically binding to GH or antigen-binding fragment thereof, the EK protease recognition sequence, the polypeptide as set forth in SEQ ID NO: 41, and the optional His tag;
preferably, the polypeptide construct comprises an amino acid sequence selected from the group consisting of:
(1) the amino acid sequence as set forth in SEQ ID NO: 14, or the amino acid sequence as set forth in positions 1-136, or positions 2-142, or positions 2-136 of SEQ ID NO: 14;
(2) the amino acid sequence as set forth in SEQ ID NO: 18, or the amino acid sequence as set forth in positions 1-136, or positions 2-142, or positions 2-136 of SEQ ID NO: 18;
(3) the amino acid sequence as set forth in SEQ ID NO: 22, or the amino acid sequence as set forth in positions 1-136, or positions 2-142, or positions 2-136 of SEQ ID NO: 22;
(4) the amino acid sequence as set forth in SEQ ID NO: 26, or the amino acid sequence as set forth in positions 1-136, or positions 2-142, or positions 2-136 of SEQ ID NO: 26;
(5) the amino acid sequence as set forth in SEQ ID NO: 28, or the amino acid sequence as set forth in positions 1-136, or positions 2-142, or positions 2-136 of SEQ ID NO: 28;
(6) the amino acid sequence as set forth in SEQ ID NO: 30, or the amino acid sequence as set forth in positions 1-136, or positions 2-142, or positions 2-136 of SEQ ID NO: 30;
(7) the amino acid sequence as set forth in SEQ ID NO: 32, or the amino acid sequence as set forth in positions 1-136, or positions 2-142, or positions 2-136 of SEQ ID NO: 32;
(8) the amino acid sequence as set forth in SEQ ID NO: 33, or the amino acid sequence as set forth in positions 1-136, or positions 2-142, or positions 2-136 of SEQ ID NO: 33;
(9) the amino acid sequence as set forth in SEQ ID NO: 35, or the amino acid sequence as set forth in positions 1-136, or positions 2-142, or positions 2-136 of SEQ ID NO: 35;
(10) the amino acid sequence as set forth in SEQ ID NO: 36, or the amino acid sequence as set forth in positions 1-136, or positions 2-142, or positions 2-136 of SEQ ID NO: 36.

11. An isolated nucleic acid molecule encoding the single-domain antibody or antigen-binding fragment thereof of any one of claims 1 to 6, or the polypeptide construct of any one of claims 7 to 10.

12. A vector, comprising the isolated nucleic acid molecule of claim 11, wherein preferably, the vector is a cloning vector or an expression vector.

13. A host cell, comprising the isolated nucleic acid molecule of claim 11 or the vector of claim 12.

14. A method for preparing the single-domain antibody or antigen-binding fragment thereof of any one of claims 1 to 6, or the polypeptide construct of any one of claims 7 to 10, comprising culturing the host cell of claim 13 under conditions permitting protein expression, and recovering the single-domain antibody or antigen-binding fragment thereof or the polypeptide construct from the culture of the cultured host cells.

15. A conjugate, comprising the single-domain antibody or antigen-binding fragment thereof of any one of claims 1 to 6, or the polypeptide construct of any one of claims 7 to 10, and a solid support linked to the single-domain antibody or antigen-binding fragment thereof or the polypeptide construct;
preferably, the solid support is selected from: magnetic beads, agarose microspheres, polymeric matrices (e.g., dextran microspheres, polymethacrylate, polystyrene, polystyrene-divinylbenzene, poly(methyl propionate)), silica matrices (e.g., silica microspheres), graphitized carbon matrices, alumina matrices, zirconia matrices, and any combination thereof;
preferably, the solid support is selected from porous materials, for example, a single porous material or a combination of multiple porous materials.

16. A method for purifying GH, comprising using the conjugate of claim 15;
preferably, the method is a method for purifying GH by affinity chromatography;
preferably, the method comprises the following steps:
(1) allowing the conjugate to bind to the GH in a first solvent to form a complex;
(2) dissociating the complex in a second solvent; and
(3) collecting the dissociated product containing the GH;
wherein the first solvent is a solvent suitable for forming a complex between the conjugate and the GH, and the second solvent is a solvent capable of dissociating the complex.

17. Use of the single-domain antibody or antigen-binding fragment of any one of claims 1 to 6, or the polypeptide construct of any one of claims 7 to 10, or the conjugate of claim 15, in the preparation of a reagent for GH purification.

18. A conjugate, comprising the single-domain antibody or antigen-binding fragment thereof of any one of claims 1 to 6, or the polypeptide construct of any one of claims 7 to 10, and a detectable label linked to the single-domain antibody or antigen-binding fragment thereof or the polypeptide construct;
preferably, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

19. A kit, comprising the single-domain antibody or antigen-binding fragment of any one of claims 1 to 6, or the polypeptide construct of any one of claims 7 to 10, or the conjugate of claim 18;
preferably, the kit comprises the conjugate of claim 18;
preferably, the kit comprises the single-domain antibody or antigen-binding fragment thereof of any one of claims 1 to 6, or the polypeptide construct of any one of claims 7 to 10, and a secondary antibody that specifically recognizes the single-domain antibody or antigen-binding fragment thereof or the polypeptide construct; optionally, the secondary antibody further comprises a detectable label, such as an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

20. A method for detecting the presence or level of GH in a sample, comprising using the single-domain antibody or antigen-binding fragment of any one of claims 1 to 6, or the polypeptide construct of any one of claims 7 to 10, or the conjugate of claim 18;
preferably, the method is an immunological assay, such as an immunoblot, an enzyme immunoassay (e.g., ELISA), a chemiluminescent immunoassay, a fluorescent immunoassay, or a radioimmunoassay;
preferably, the method comprises using the conjugate of claim 18;
preferably, the method comprises using the single-domain antibody or antigen-binding fragment of any one of claims 1 to 6, or the polypeptide construct of any one of claims 7 to 10, and the method further comprising using a secondary antibody carrying a detectable label (e.g., an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin) to detect the single-domain antibody or antigen-binding fragment or the polypeptide construct.

21. Use of the single-domain antibody or antigen-binding fragment thereof of any one of claims 1 to 6, or the polypeptide construct of any one of claims 7 to 10, or the conjugate of claim 18, in the preparation of a detection reagent, wherein the detection reagent is used for detecting the presence or level of GH in a sample and/or diagnosing diseases associated with abnormal GH levels;
preferably, the detection reagent is used to detect the presence or level of GH in a sample by the method of claim 20;
preferably, the sample is a body fluid sample from a subject (e.g., a mammal, preferably a human).
